# EUROPEAN PATENT SPECIFICATION

(11) **EP 1 572 672 B1**
(45) Date of publication and mention of the grant of the patent: **13.08.2008**
(21) Application number: 03786172.1
(22) Date of filing: 22.12.2003
(51) Int. Cl.: C07D 267/22, C07D 413/04, A61K 31/395, A61K 31/422, A61P 29/00, C07D 498/04

(54) **BENZOXAZOCINES AND THEIR USE AS MONOAMINE-REUPTAKE INHIBITORS**
BENZOXAZOCINE UND IHRE VERWENDUNG ALS MONOAMIN-WIEDERAUFNAHME INHIBITOREN
BENZOXAZOCINES ET UTILISATION DE CEUX-CI COMME INHIBITEURS DE LA RECAPTURE DES MONO-AMINES

(30) Priority: 20.12.2002 GB 0229743; 18.07.2003 GB 0316914
(43) Date of publication of application: 14.09.2005
(60) Divisional application: 08102734.4
(73) Proprietor: Sosei R&D Ltd., Little Chesterford Saffron Walden Essex CB10 1XL (GB)
(72) Inventor: BAXTER, Andrew, Douglas Arakis Ltd., Saffron Walden EsseLittle Chesterford Sa (GB)
(74) Representative: Perry, Robert Edward
(86) International application number: PCT/GB2003/005708
(87) International publication number: WO 2004/056788

(56) References cited:
- GB-A- 1 148 717
- US-A- 3 978 085
- ROSLAND J H ET AL: "The effect of nefopam and its enantiomers on the uptake of 5-hydroxytryptamine, noradrenaline and dopamine in crude rat brain synaptosomal preparations" JOURNAL OF PHARMACY AND PHARMACOLOGY, vol. 42, no. 6, June 1990 (1990-06), pages 437-438, XP008029309

## Description

This invention relates to novel benzoxazocine compounds which inhibit monoamine reuptake and their therapeutic use.

### Background of the Invention

Nefopam [(±)-3,4,5,6-tetrahydro-5-methyl-1-phenyl-1H-2,5-benzoxazocine hydrochloride] is a centrally acting non-narcotic analgesic not structurally related to other analgesics. Nefopam has been shown to induce antinociception in animal models of pain and in humans (reviewed in Heel *et al.,* 1980). However, nefopam is not active in the mouse tail-flick test, or the hot plate test and the Randall-Selitto pressure test in rats (Conway and Mitchell, 1977) suggesting that its analgesic mechanism is not opiate-like or anti-inflammatory in nature. Nefopam's antinociception is not blocked by nalaxone further suggesting that its analgesic action is not through opiate receptors. Although the precise, mechanism of antinociception is not known it is thought to involve inhibition of synaptosomal uptake of dopamine, norepinephrine and serotonin (VonVoigtlander *et al.,* 1983; Rosland and Hole, 1990; Mather *et al.,* 2001). Previous *in vitro* and *in vivo* studies with nefopam enantiomers have shown that (+)-nefopam has more potent analgesic and dopamine, norepinephrine and serotonin uptake inhibitory properties than (-)-nefopam with the order of potency given as (+)-nefopam > (±)-nefopam > (-)-nefopam (Fasmer *et al*., 1987; Rosland and Hole, 1990; Mather *et al.,* 2001).

GB1148717 discloses nefopam, N-substituted analogues and also analogues wherein the benzenoid portion of the fused ring structure and/or the phenyl radical bears one or more lower alkyl, lower alkoxy, halogen or CF₃ substituents.

US3978085 discloses halogen-substituted nefopam analogues, as intermediates.

J. Pharm. Pharmacol. 1990, 42: 437-438, describes the inhibition of amine uptake by nefopam.

### Summary of the Invention

According to this invention, novel compounds are of general formula (1): 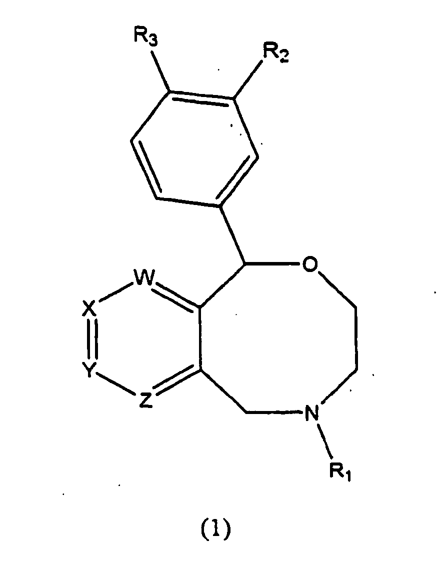 wherein
R₁ is H, C₁-C₆alkyl, optionally substituted with F or C₃-C₆ cycloalkyl or C₂-C₆ alkenyl;
either R₂ and R₃ are the same or different and are H (but are not both H), a halogen, CN, CF₃, C₁-C₆alkyl or OR₁, or R₂ and R₃ form a five or six membered ring which may be carbocyclic, heterocyclic (containing 1-2 heteroatoms taken from 0, N or S), aromatic (such as in naphthalene for example), heteroaromatic (containing 1-2 heteroatoms taken from O, such as in benzofuran for example, N as in quinoline, isoquinoline and quinazoline for example); and
W, X, Y or Z are each N, CH or CR₄.

The case where W = X = Y = Z = CH is specifically excluded. When W is N or R₄, X=Y=Z=CH; when X is N or R₄, W = Y = Z = CH; when Y is N or R₄, W = X = Z = CH; and when Z is N or R₄, W = X = Y = CH.

R₄ is halogen, CF₃, CN, OR₇, SO₂N(R₆)₂ (where each R₆ is the same or different), COR₆, CO₂R₆, CON(R₆)₂ (where R₆ maybe the same or different), NR₁COR₅, NR₁SO₂R₅, NR₁CO₂R₅, NR₁CON(R₆)₂ (where each R₆ is the same or different), OC₁-C₆ alkyl optionally substituted with R₄, C₁-C₆ alkyl optionally substituted with R₄, C₃-C₆ cycloalkyl optionally substituted with R₄, C₂-C₆ alkenyl optionally substituted with R₄ , C₂-C₆ alkynyl optionally substituted with R₄, and aryl optionally substituted with R₄. R₄ may also be a five or six membered aromatic heterocycle containing 1-4 heteroatoms selected from N (such as in pyrrole, pyridine, diazoles, diazines, triazoles, triazines or tetrazoles for example) and O (such as in furan, oxazoles, isoxazoles or oxadiazoles for example). Such rings can be linked either through carbon or nitrogen.

R₅ is C₁-C₆alkyl, C₂-C₆alkenyl, C₂-C₆ alkynyl, C₃-C₆cycloalkyl, aryl or heteroaryl.

R₆ is H, C₁-C₆alkyl, C₂-C₆alkenyl, C₂-C₆ alkynyl, C₃-C₆cycloalkyl, aryl or heteroaryl.

R₇ is aryl or heteroaryl.

Salts, solvates and polymorphs of these compounds are also included.

Compounds of the invention are useful as therapeutic agents. Further, in compounds of formula (1), those wherein R₄ is a halogen atom such as Br are useful as intermediates.

### Description of Preferred Embodiments

It will be appreciated that the compounds according to the invention contain an asymmetrically substituted carbon atom. The presence of this asymmetric centre in a compound of formula (1) can give rise to stereoisomers, and in each case the invention is to be understood to extend to all such stereoisomers, including enantiomers and diastereomers, and mixtures including racemic and non-racemic mixtures thereof.

As used in this specification, alone or in combination, the term "C₁-C₆ alkyl" refers to straight or branched chain alkyl moiety having from one to six carbon atoms, including for example, methyl, ethyl, propyl, isopropyl, butyl, *tert-*butyl, pentyl, hexyl and the like.

The term "C₂-C₆ alkenyl" refers to a straight or branched chain alkyl moiety having two to six carbon atoms and having in addition one double bond, of either E or Z stereochemistry where applicable. This term would include for example, vinyl, 1-propenyl, 1- and 2- butenyl, 2- methyl-2-propenyl etc.

The term "C₂-C₆ alkynyl" refers to a straight or branched chain alkyl moiety having two to six carbon atoms and having in addition one triple bond. This term would include for example, ethynyl, 1-propargyl, 1- and 2- butynyl etc.

The term "C₃-C₆ cycloalkyl" refers to a saturated alicyclic moiety having from three to six carbon atoms and includes for example cyclopropyl, cyclobutyl, cyclopentyl, cyclohexyl and the like.

The term "aryl" means an optionally substituted phenyl or naphthyl group.

The term "carbocyclic" refers to a saturated alicyclic moiety having five or six carbon atoms and includes for example benzofused cyclopentyl and cyclohexyl and the like.

The term "heterocyclic" refers to a saturated heterocyclic moiety having from five or six atoms but containing one or more heteroatom from the group N, O, S and includes for example benzofused pyrrolidinyl, tetrahydrofuranyl, piperidinyl, dioxalane and the like.

The term "heteroaromatic" refers to aromatic ring systems of five or six atoms or which at least one atom is selected from the group, O, N, or S and includes for example benzofused furanyl, thiophenyl, pyridyl, indolyl, pyridazinyl, piperazinyl, pyrimidinyl and the like.

The term "halogen" means fluorine, chlorine, bromine or iodine.

Compounds of the general formula (1) may be prepared by any suitable method known in the art and/or by the processes described below. It will be appreciated that where a particular stereoisomer of formula (1) is required, the synthetic processes described herein may be used with the appropriate homochiral starting material and/or isomers maybe resolved from mixtures using conventional separation techniques (e.g. HPLC).

The compounds according to the invention may be prepared by the following process. In the description and formulae below the groups R₁, R₂, R₃, R₄, R₅, R₆, W, X, Y and Z are as defined above, except where otherwise indicated. It will be appreciated that functional groups, such as amino, hydroxyl or carboxyl groups, present in the various compounds described below, and which it is desired to retain, may need to be in protected form before any reaction is initiated. In such instances, removal of the protecting group may be the final step in a particular reaction. Suitable protecting groups for such functionality will be apparent to those skilled in the art. For specific details see "Protective Groups in Organic Synthesis", Wiley Interscience, T W Greene, PGM Wuts.

A process required for preparing compounds of general formula (1), where W, X, Y or Z are N or C-Br comprises acid (for instance p-toluenesulphonic acid) cyclisation of the diol of general formula (2) which can in turn be obtained by reduction of the ketone (3) with a suitable reducing agent.

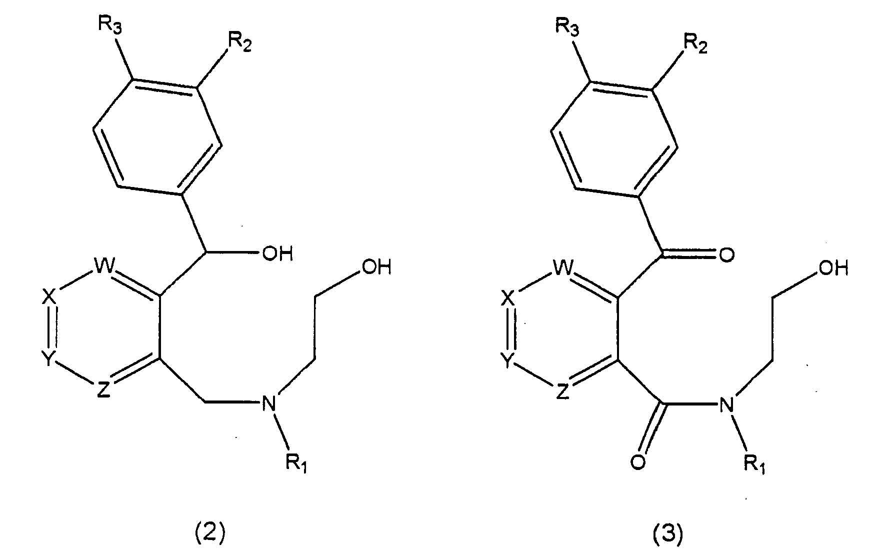

Reduction of a keto amide of general formula (3) can be carried out with reagents well known to those familiar in the art of synthetic organic chemistry. An example of a highly reactive reducing agent is lithium aluminium hydride, although reagents based on borane (e.g. borane.tetrahydrofuran complex) or modified sodium borohydride reduction (e.g. with a nickel or cobalt salt enhancer) are equally effective.

Equally, reduction of the ketone in (3), for example with sodium borohydride, followed by acid cyclisation, for example with p-toluenesulphonic acid, then ultimate reduction of the amide group, for example with borane, also leads to compounds of general formula (1).

Ketones of general formula (3) can be prepared by condensation of a carboxylic acid of general formula (4) or an active derivative thereof, with an amine of formula (5). Active derivatives of acids of formula (4) include for example acid anhydrides or acid halides, such as acid chlorides.

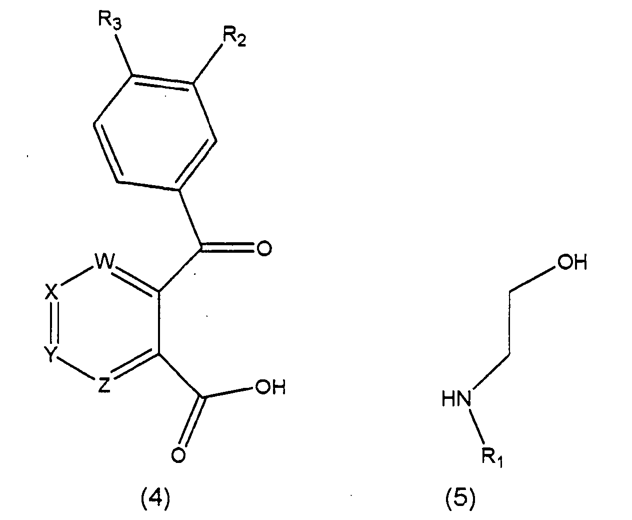

The coupling reaction may be performed using standard conditions for amidation reactions of this type. Thus, the reaction may be achieved in a solvent, for example an inert organic solvent such as an ether, e.g. a cyclic ether such as tetrahydrofuran, an amide e.g. a substituted amide such as dimethylformamide, or a halogenated hydrocarbon such as dichloromethane at a low temperature e.g. -30°C to ambient temperature, such as -20°C to 0°C, optionally in the presence of as base, e.g. an organic base such as an amine, e.g. triethylamine or a cyclic amine such as N-methylmorpholine. Where an acid of formula (4) is used directly, the reaction may additionally be performed in the presence of a condensing agent, for example a diimide such as *N*,*N*'-dicyclohexylcarbodiimide, advantageously in the presence of a triazole such as 1-hydroxybenzotriazole. Alternatively, the acid may be reacted with a chloroformate, for example ethyl chloroformate, prior to reaction with the amine of formula (5).

Acids of general formula (4) are prepared by Friedel-Crafts acylation of an arene of general formula (6) with an anhydride of formula (7). This reaction is carried out in an inert solvent (such as dichloromethane) in the presence of a Lewis acid catalyst (such as aluminium trichloride).

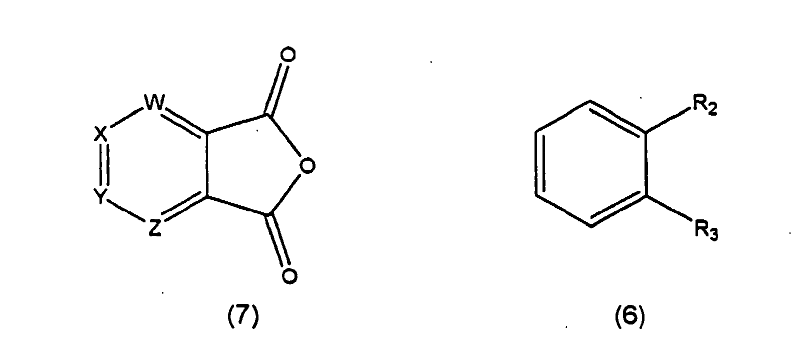

It is well recognised by those skilled in the art that such reactions may provide mixtures of products and in turn that these mixtures can often be separated by traditional flash column chromatography.

Compounds of general formula (1) where either W, X, Y or Z is CR₄ and R₄ is a halogen such as Br (1 a) represent flexible intermediates that may be used for the preparation of other compounds of general formula (1). For instance, compounds of general formula (1) where either W, X, Y or Z is CR₄ and R₄ is Br can be smoothly converted into the corresponding nitrile (1b; R₄ = CN) either by reaction with cuprous cyanide in a dipolar aprotic solvent such as *N-*methylpyrrolidinone (NMP) or under palladium-catalysed conditions (Scheme 1).

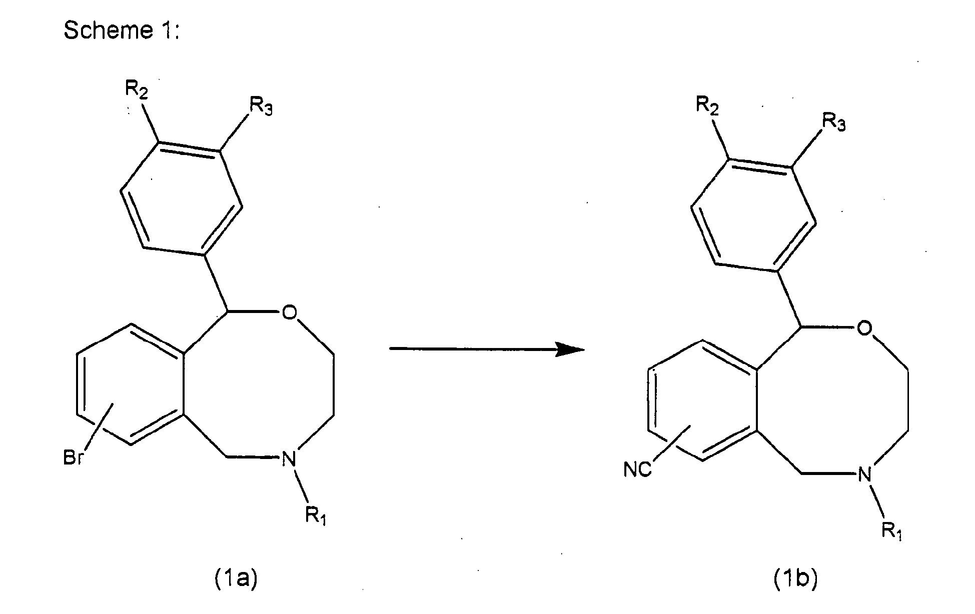

The nitrile of general formula (1) where either W, X, Y or Z is CR₄ and R₄ is CN (1 b) can be readily converted, by hydrolysis, into the primary amide (1 c, R₄ = CONH₂), esters and the corresponding carboxylic acid (1d, CO₂R₁) or into the corresponding tetrazole (1e) by treatment with a suitable azide donor such as sodium azide or trimethylsilylazide (Scheme 2).

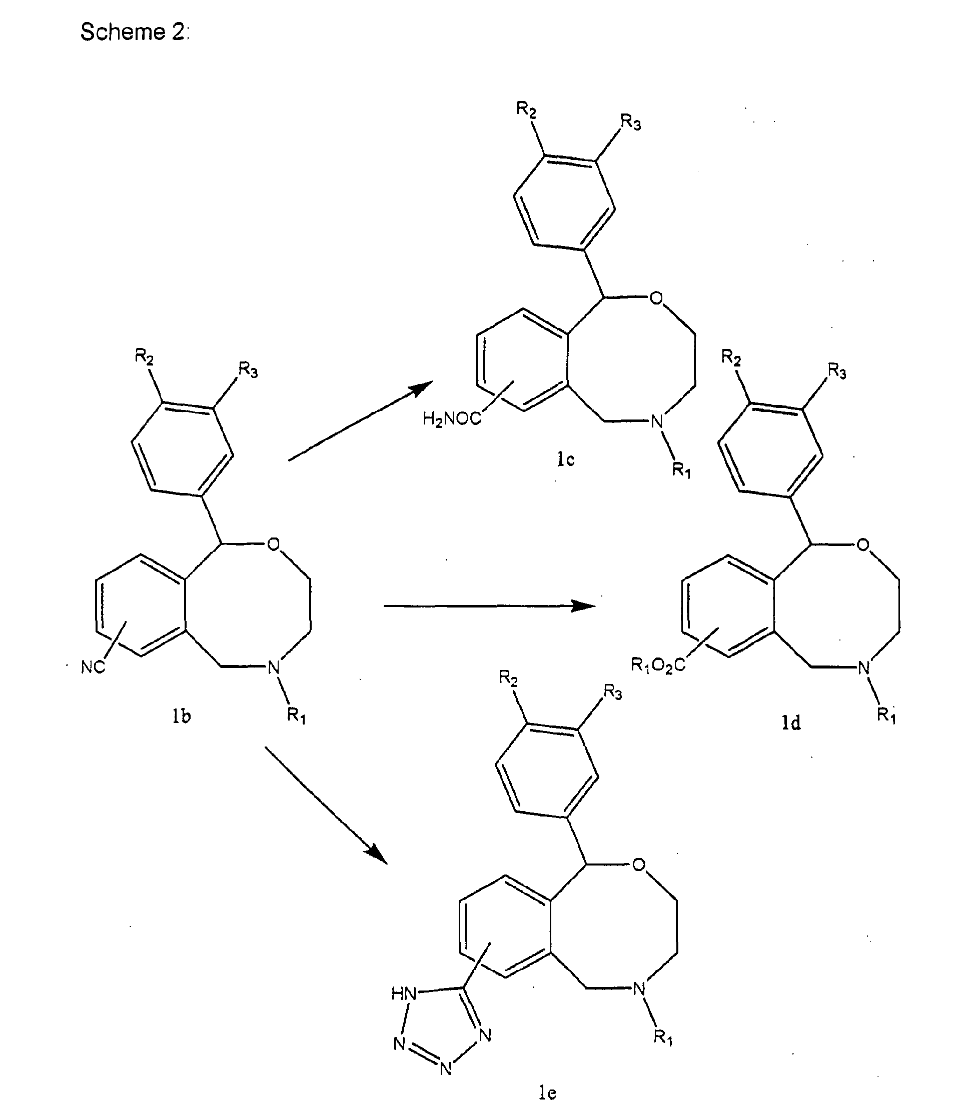

In addition, compounds of general formula (1) where either W, X, Y or Z is CR₄ and R₄ is a halogen such as Br (1a) can be lithiated with n-, sec-, or *tert-*butyllithium in an inert organic solvent such as an ether, e.g. a cyclic ether such as tetrahydrofuran at very low temperature, e.g. -78°C. Treatment with either a carbon (e.g. carbon dioxide, *N*,*N*-dimethyl formamide or paraformaldehyde), sulphur (e.g. SO₂Cl₂, followed by amidation, such as with ammonia) or nitrogen (diphenylphosphoryl azide, followed by reduction, such as with REDAL) provides access, by subsequent derivatisation to derivatives where R₄ is CO₂R₁; CON(R₁)₂ (where each R₁ is the same or different); CH₂OR₁ (1f), SO₂N(R₁)₂ (1g, where each R₁ is the same or different); and NR₁COR₅; NR₁SO₂R₅ (1h); NR₁CO₂R₅; NR₁CON(R₁)₂ (where each R₁ is the same or different). Examples are given in Scheme 3.

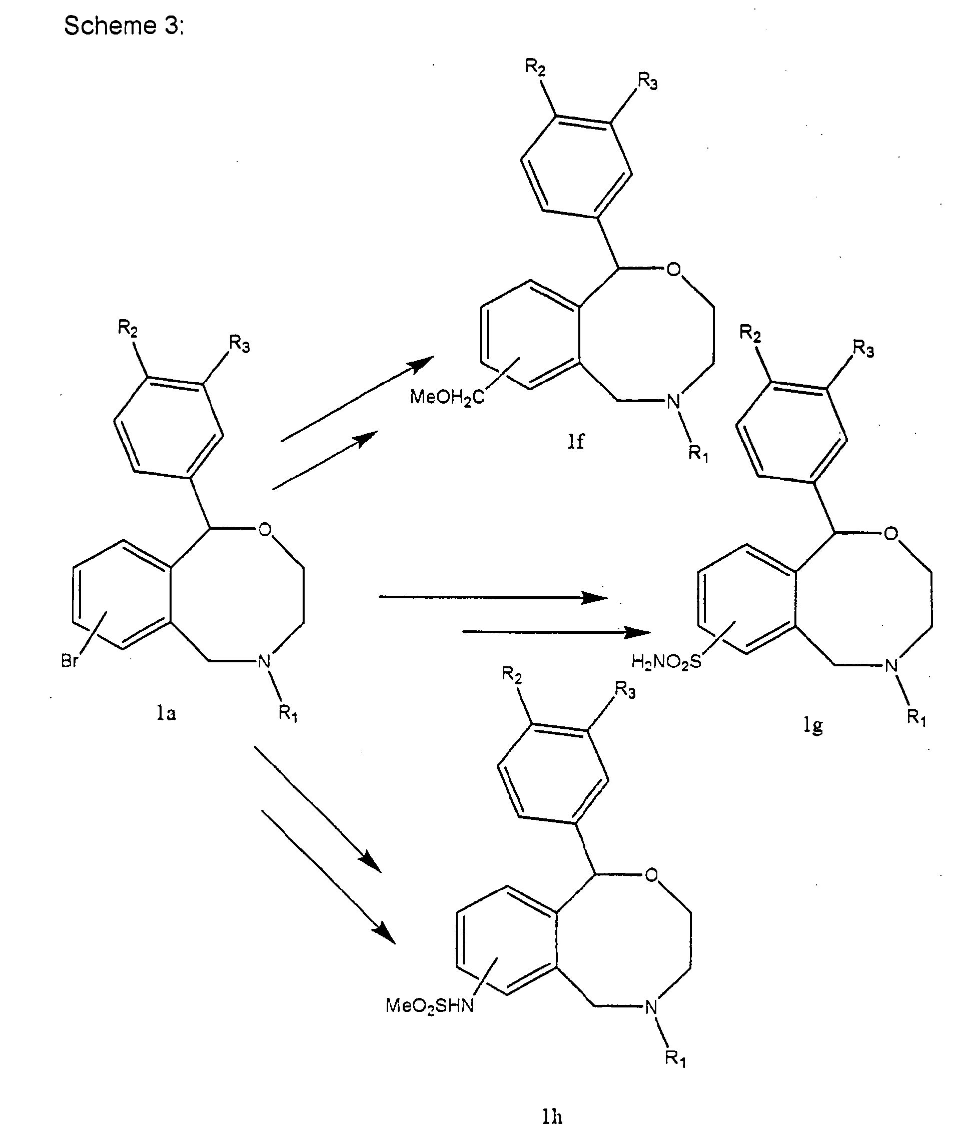

In addition, compounds of general formula (1) where either W, X, Y or Z is CR₄ and R₄ is a halogen such as Br (1a) can undergo palladium-catalysed coupling reactions with carbon-based coupling partners. Thus, compounds of general formula (1) where either W, X, Y or Z is CR₄ and R₄ is a halogen such as Br can be coupled to alkenes of a general type CH₂=CHR₄ under Heck conditions, alkynes of a general type CH=CHR₄ under Sonogoshira conditions, or metalloheterocycles e.g. where the metal is tin, under Stille coupling conditions. This gives access to compounds where either W, X, Y or Z can be C₂-C₆ alkenyl substituted with R₄ (1i) C₂-C₆ alkynyl substituted with R₄ (1j) and where R₄ is a five membered aromatic heterocycle containing 1-4 heteroatoms taken from N (such as in pyrrole, diazoles, triazoles or tetrazoles for example) and O (such as in furan (1k), oxazoles or oxadiazoles for example). Such coupling reactions ensure that chains and rings are linked either through carbon. Examples are in Scheme 4.

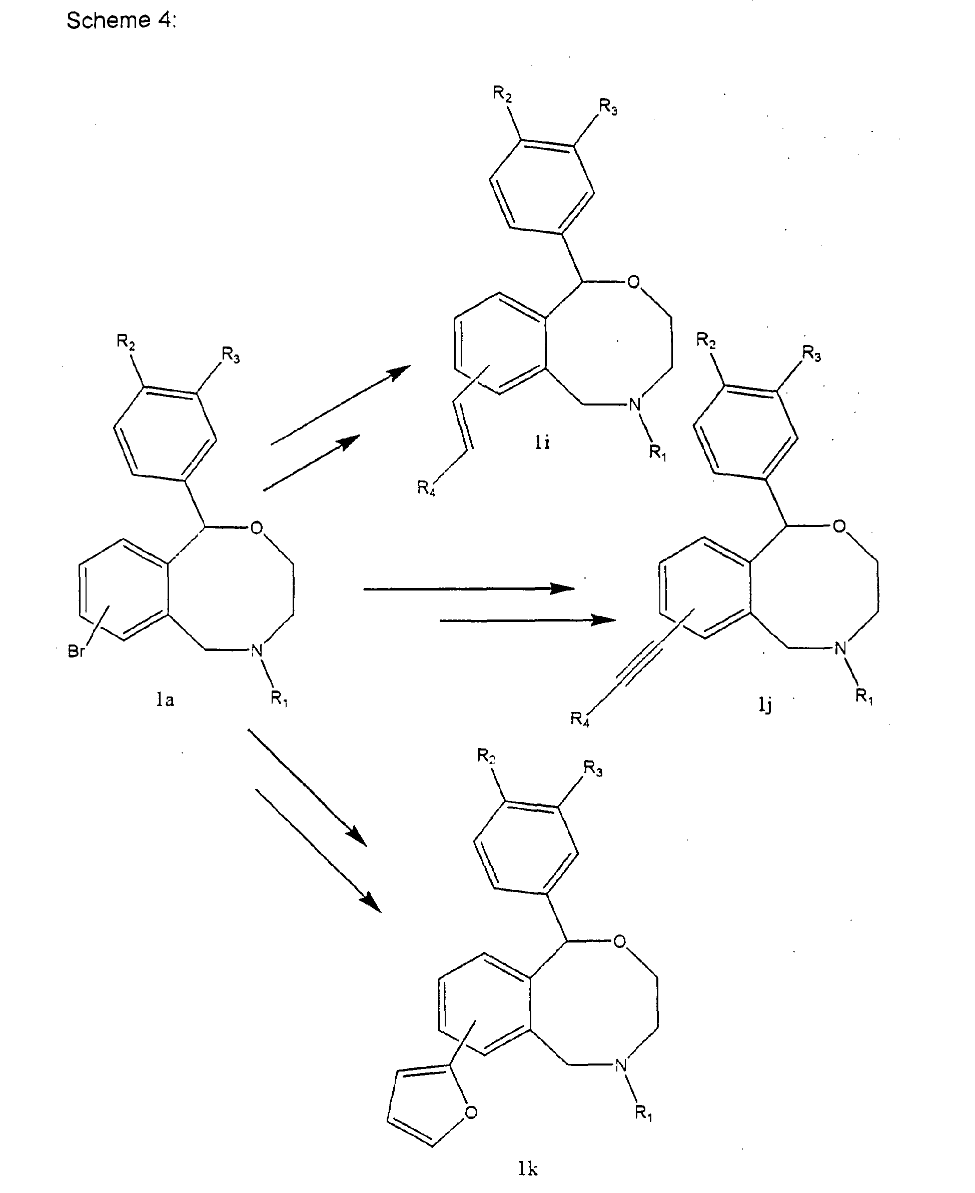

In addition to the examples described above, additional compounds of formula (1) may be prepared by interconversion of other compounds of formula (1). Thus, for example, a compound of formula (1l) wherein R₄ is a C₁₋₆ alkyl group may be prepared by hydrogenation (using palladium on carbon in suitable solvent, such as an alcohol- e.g. ethanol) of a compound of formula (1i) wherein R₄ is a C₂₋₆ alkenyl group (e.g. as in Scheme 5).

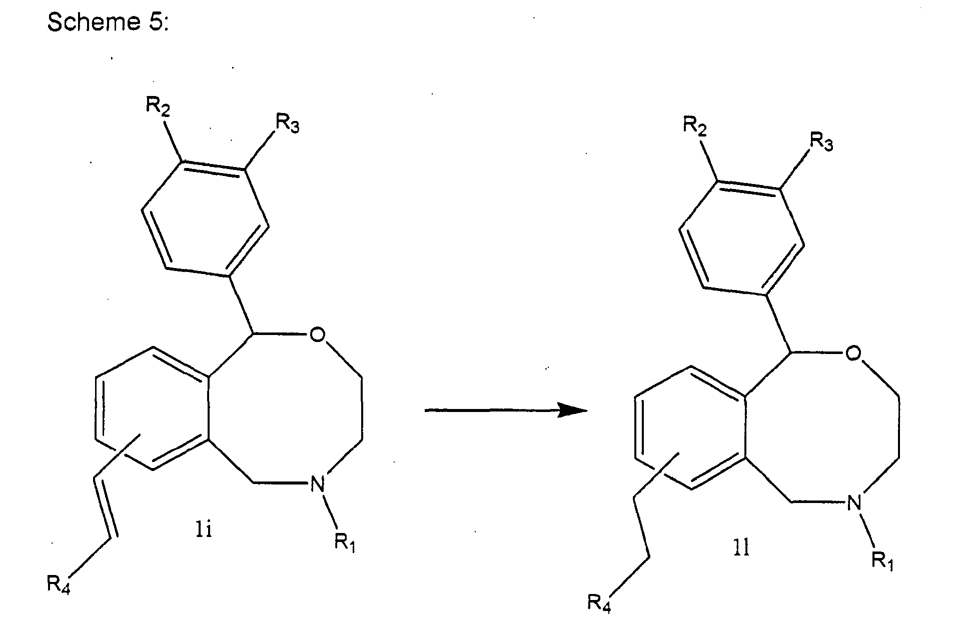

Any mixtures of final products or intermediates obtained can be separated on the basis of the physico-chemical differences of the constituents, in known manner, into the pure final products or intermediates, for example by chromatography, distillation, fractional crystallization, or by formation of a salt if appropriate or possible under the circumstances.

The compounds according to the invention exhibit *in vitro* inhibiting activities with respect to monoamine (i.e. noradrenaline, serotonin and dopamine) reuptake. The activity and selectivity of the compounds may be determined by use of an appropriate monoamine reuptake assay.

This invention also relates to a method of treatment for patients (including man and/or mammalian animals raised in the dairy, meat or fur industries or as pets) suffering from disorders or diseases which can be attributed to monoamine reuptake as previously described, and more specifically, a method of treatment involving the administration of the monoamine reuptake inhibitor of formula (1) as the active constituents.

Accordingly, the compounds of formula (1) can be used among other things in the treatment of pain and emesis but also may find utility in a range of other therapeutic indications such as depression, post-traumatic stress disorders, attention-deficit disorders, obsessive compulsive disorders, pre-menstrual syndrome, substance abuse and sexual dysfunction.

Compunds of formula (1) may be administered orally, topically, buccally, ocularly, rectally, vaginally, parenterally, intra-nasally, sublingually or by inhalation spray, e.g. in dosage unit formulations containing non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle, sheep, dogs, cats etc, the compounds of the invention are effective in the treatment of humans.

The pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. The composition may be in immediate or controlled release form.

Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyceryl distearate may be employed. They may also be coated by the techniques described in the US Patents 4,256,108;4,166,452; and 4,265,874 to form osmotic therapeutic tablets for control release.

Formulations for oral use may also be presented as hard gelatin capsules where in the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate polyvinylpyrrolidone, gum tragacanth and gum acacia; dispersing or wetting agents may be a naturally occuring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters dervied from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents maybe added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified, for example sweetening, flavouring and colouring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally occurring gums, for example gum acacia or gum tragacanth, naturally-occuring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example gycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be in a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables,

The compounds of formula (1) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc containing the compounds of Formula (1) are employed. For the purposes of this application, topical application includes mouth washes and gargles.

Dosage levels of the order of from about 0.05 mg to about 140 mg per kilogram of body weight per day are useful in the treatment of the above-indicated conditions (about 2.5 mg to about 7 gms per patient per day). For example, emesis may be effectively treated by the administration of from about 0.01 to 50 mg of the compound per kilogram of body weight per day (about 0.5 mg to about 3.5 gms per patient per day).

The amount of active ingredient that may be combined with the carrier materials to produce a single dosage form will vary depending upon the host treated and the particular mode of administration. For example, a formulation intended for the oral administration of humans may vary from about 5 to about 95 percent of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy.

The following Examples illustrate the invention.

### 2-(4-Methoxy)benzoyl-4-bromobenzoic acid and 2-(4-methoxy)benzoyl-5-bromobenzoic acid (14)

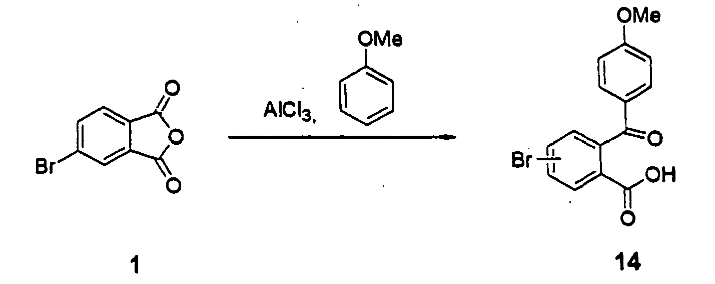

To a flask containing 4-bromophthalic anhydride (1) (25.65g, 0.11mol) was added finely crushed aluminium chloride (30.13g, 0.23mol). The solid mixture was further crushed and stirred using a spatula. To this mixture was added anisole (85.53g, 86ml, 0.79mol) which initiated the production of HCl gas. Once gas evolution ceased the reaction was heated at 80°C for 1.5 hours. The hot reaction mixture was poured into a solution of water : conc. hydrochloride acid (9:1, 600mL) and the aqueous layer extracted with ether (2 x 150mL). The organic layer was washed with water and then brine before being dried over magnesium sulphate, filtered and evaporated under reduced pressure to furnish the crude product. Overnight recrystallisation from a mixture of ether (200ml) and hexane (400ml) yielded **14,** after filtration, a white solid as a mixture of regio isomers. Yield 22.77g, 60%.

¹H nmr (250MHz, CDCl₃); 9.28 (2H, brs, 2xCO₂H), 8.19 (1H, d, *J* 1.8, CHₐᵣ), 7.92 (1 H, d, *J* 8.4, CHₐᵣ), 7.78 (1 H, d, *J* 1.8, CHₐᵣ), 7.74 (1 H, d, *J* 1.8, CHₐᵣ), 7.69 (2H, d, *J* 2.0, CHₐᵣ), 7.65 (2H, d, *J* 1.8, CHₐᵣ), 7.48 (1 H, d, *J* 1.8, CHₐᵣ). 7.22 (1 H, d, *J* 8.1 CHₐᵣ), 6.91 (2H, d, *J* 2.5, CHₐᵣ), 6.88 (2H, d, *J* 2.5, CHₐᵣ), 3.86 (6H, s, 2xCH₃).

### N-(2-hydroxyethyl)-N-methyl-2-(4-methoxy)benzoyl-4-bromobenzamide (15a) and N-(2-hydroxyethyl)-N-methyl- 2-(4-methoxy)benzoyl-5-bromobenzamide (15b)

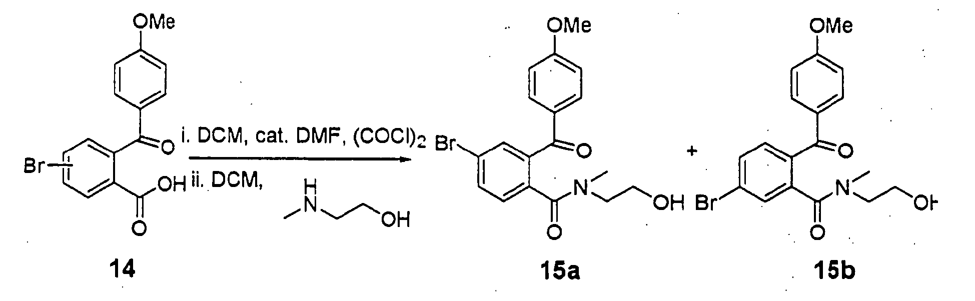

A solution of 2M oxalyl chloride (6.52mL, 74.73mmol, 1.1 equiv.) in dichloromethane was added dropwise to a suspension of the mixture of isomers **14** (22.77g, 67.94mmol) in dichloromethane (115mL) and catalytic *N,N-*dimethylformamide (5 drops) at room temperature under a nitrogen atmosphere. Gas evolution was rapid and as the reaction proceeded the solid dissolved in the dichloromethane. After 2.5 hours the solvent was removed under reduced pressure and the resulting solid co-evaporated with dichloromethane (2 x 100mL) to remove traces of excess oxalyl chloride. The crude acid chloride was dissolved in dichloromethane (115mL) and added dropwise to a solution of *N-*methylaminoethanol (6.0mL, 74.73mmol, 1.1 equiv.) and triethylamine, (10.42mL, 74.73mmol, 1.1 equiv) in dichloromethane (115mL) cooled to 0°C in an ice bath. The resulting solution was stirred at room temperature for 3 hours, quenched with saturated aqueous sodium dicarbonate (100mL) and separated. The aqueous layer was extracted with dichloromethane (100mL) and the combined organic fractions washed with brine (50mL), dried over magnesium sulfate and filtered. The solvent was removed under reduced pressure and the crude product purified by column chromatography, eluting with ethyl acetate : hexane (4:1), followed by ethyl acetate (100%) as product eluted. Compound 15a was furnished as a yellow semi solid. Yield 5.00g, 19%. The product exists are a mixture of rotomers in a 3:2 ratio.

¹H nmr (250MHz, CDCl₃); 7.81 (2H, d, *J* 7.5, CHₐᵣ), 7.71-7.53 (2H, m, CHₐᵣ), 7.31 (1 H, m, CHₐᵣ), 6.95 (2H, d, *J* 7.6, CHₐᵣ), 3.88 (3H, s, OCH₃), 3.80 (2H, t, *J* 5.1, CH₂OH), 3.72 (1H, m OH), 3.57 (2H, t, *J* 4.9, NCH₂), 3.06 (1.2H, s, NCH₃) 2.96 (1.8H, s, NCH₃).

Compound **15b** was furnished as a red semi solid. Yield 5.00g, 19%. The products exist as a mixture of rotomers in a 3:2 ratio.

¹H nmr (250MHz, CDCl₃); 7.77 (2H, dd, *J* 9.0, 2.4, CHₐᵣ), 7.59-7.53 (2H, m, CHₐᵣ), 7.42-7.31 (1H, m, CHₐᵣ), 6.91 (2H, dd, *J* 9.0, 2.4, CHₐᵣ), 3.85 (3H, s, OCH₃), 3.79 (2H, t, *J* 5.1, CH₂OH), 3.69 (1 H, brs OH), 3.56 (2H, t, *J* 5.1, NCH₂), 3.03 (1.2H, s, NCH₃) 2.95 (1.8H, s, NCH₃).

### N-(2-hydroxyethyl)-N-methyl-4-bromo-2-[1-hydroxy-1-(4-methoxyphenyl)] methylbenzylamine (16a)

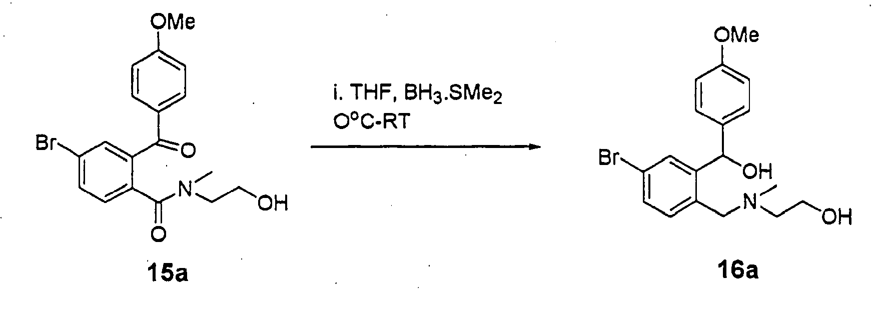

Amide **15a** (1.56g, 3.99mmol) was dissolved in tetrahydrofuran (10mL) and cooled to 0°C in an ice bath. A 2M solution of borane dimethylsulfide complex (8.78mL, 17.55mmol, 4.4 equiv.) was added dropwise and the resulting solution stirred at room temperature for 17 hours. The reaction was carefully quenched with 2M hydrochloric acid solution (5mL) and the resulting solution stirred at room temperature for 2 hours. Tetrahydrofuran was removed under reduced pressure and remaining solution diluted with water (10mL) and extracted with diethyl ether (2 x 50mL). The aqueous layer was basified with 3.75M sodium hydroxide solution and extracted with ethyl acetate (2 x 50mL). The combined ethyl acetate extracts were dried over magnesium sulfate, filtered and evaporated under reduced pressure to furnish the desired product **16a** as a white foam. Yield 754mg, 50%.

¹H nmr (250MHz, CDCl₃); 7.39-7.24 (4H, m, CHₐᵣ), 7.07-6.98 (1H, m, CHₐᵣ), 6.89-6.79 (2H, m, CHₐᵣ), 5.77 (1 H, s, CHOH), 3.80 (3H, s, OCH₃), 3.67 (2H, m, OCH₂), 3.41 (1 H, d, *J,* 12.4, ArCHₐH_{b}N), 3.28 (1 H, d, *J,* 12.7, ArCHₐH_{b}N), 2.57-2.50 (2H, m, NCH₂), 2.17 (3H, s, NCH₃).

### N-(2-hydroxyethyl)-N-methyl-5-bromo-2-[1-hydroxy-1-(4-methoxyphenyl)] methylbenzylamine (16b)

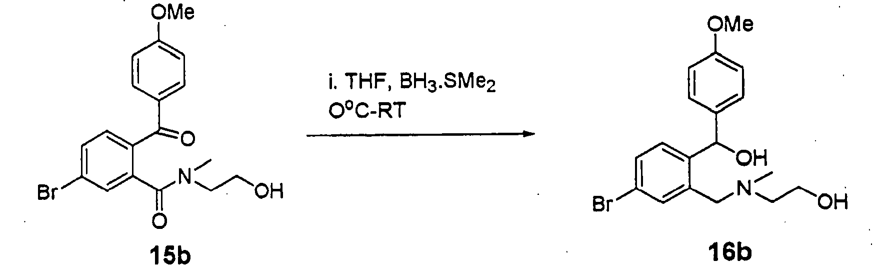

Amide **15b** (1.54g, 3.93mmol) was dissolved in tetrahydrofuran (10mL) and cooled to 0°C in an ice bath. A 2M solution of borane dimethylsulfide complex (8.60mL, 17.28mmol, 4.4 equiv.) was added dropwise and the resulting solution stirred at room temperature for 17 hours. The reaction was carefully quenched with 2M hydrochloric acid solution (5mL) and the resulting solution stirred at room temperature for 2 hours. Tetrahydrofuran was removed under reduced pressure and remaining solution diluted with water (10mL) and extracted with diethyl ether (2 x 50mL). The aqueous layer was basified with 3.75M sodium hydroxide solution and extracted with ethyl acetate (2 x 50mL). The combined ethyl acetate extracts were dried over magnesium sulfate, filtered and evaporated under reduced pressure to furnish the desired product **16b** as a white foam. Yield 606mg, 40%.

¹H nmr (250MHz, CDCl₃); 7.45-7.33 (2H, m, CHₐᵣ), 7.24 (2H, d, *J* 8.4, CHₐᵣ), 7.03-6.95 (1H, m, CHₐᵣ), 6.86 (2H, d, J 8.7, CHₐᵣ), 5.79 (1H, s, CHOH), 3.79 (3H, s, OCH₃), 3.65 (2H, m, OCH₂), 3.41 (1 H, d, J, 12.5, ArCHₐH_{b}N), 3.28 (1H, d, *J*, 12.5, ArCHₐH_{b}N), 2.56-2.50 (2H, m, NCH₂), 2.18 (3H, s, NCH₃).

### 9-Bromo-5-methyl-1-(4-methoxy)phenyl-1,3,4,6-tetrahydro-5H-benz[f]-2,5-oxazocine (17a)

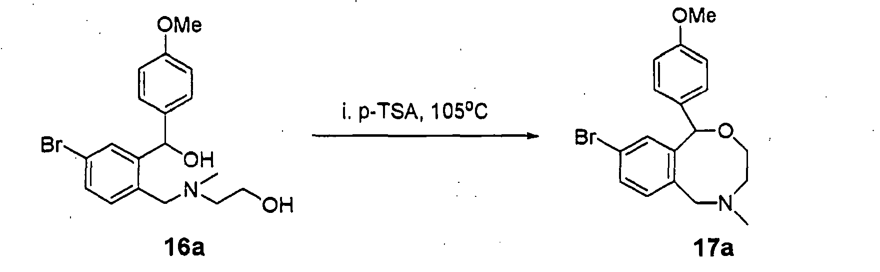

Diol **16a** (754mg, 1.99mmol) was dissolved in toluene (10mL) and paratoluenesulfonic acid monohydrate (568mg, 2.98mmol, 1.5equiv.) added. The toluene was removed under reduced pressure and the resulting oil heated at 105°C for 2 hours. On cooling the oil was suspended in water (10mL) and basified with 3.75M sodium hydroxide solution. The aqueous layer was extracted with ethyl acetate (2 x 25mL), dried over magnesium sulfate, filtered and evaporated under reduced pressure to furnish the crude product which was purified by chromatography eluting with 10% methanol in ethyl acetate. Fractions containing product were combined and evaporated under reduced pressure to furnish **17a**. Yield 340mg, 47%.

¹H nmr (250MHz, CDCl₃); 7.34 (1H, dd, J 8.1, 1.8, CHₐᵣ), 7.16 (2H, d, *J* 8.7, CHₐᵣ), 7.12-7.06 (2H, m, CHₐᵣ), 6.85 (2H, d, J 8.6, CHₐᵣ), 5.66 (1 H, s, CHOH), 4.83 (1 H, d, *J* 12.8, ArCHₐH_{b}), 4.15 (1 H, m, OCHₐH_{b}), 3.83 (1 H, OCHₐH_{b}), 3.78 (3H, s, OCH₃), 3.60 (1 H, d, *J* 12.8, ArCHₐH_{b}), 2.77 (1H, m NCHₐH_{b}), 2.59 (1H, ddd, *J* 2.6, 5.5, 14.2, NCHₐH_{b}), 2.43 (3H, s, CH₃).

### 8-Bromo-5-methyl-1-(4-methoxy)phenyl-1,3,4,6-tetrahydro-5H-benz[f]-2,5-oxazocine (17b)

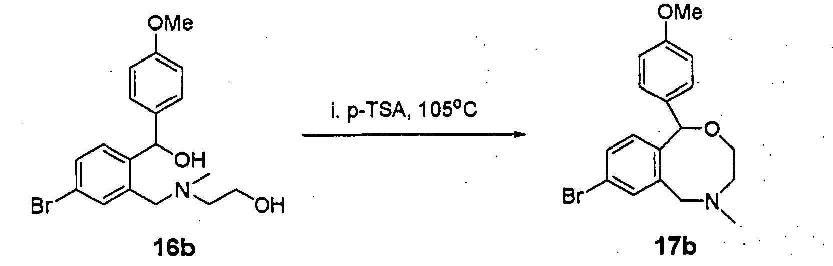

Diol **16b** (200mg, 0.53mmol) was dissolved in toluene (5mL) and paratoluenesulfonic acid monohydrate (150mg, 0.79mmol, 1.5equiv.) added. The toluene was removed under reduced pressure and the resulting oil heated at 105°C for 2 hours. On cooling the oil was suspended in water (10mL) and basified with 3.75M sodium hydroxide solution. The aqueous layer was extracted with ethyl acetate (2 x 25mL), dried over magnesium sulfate, filtered and evaporated under reduced pressure to furnish the crude product which was purified by chromatography eluting with 10% methanol in ethyl acetate. Fractions containing product were combined and evaporated under reduced pressure to furnish **17b** as a clear oil. Yield 127mg, 67%.

¹H nmr (250MHz, CDCl₃); 7.36 (1 H, d, *J* 1.5, CHₐᵣ), 7.29 (1 H, dd, *J* 8.4, 1.8, CHₐᵣ), 7.15 (2H, d, *J* 8.7, CHₐᵣ), 6.85 (1 H, m, CHₐᵣ), 6.83 (2H, d, *J* 8.6, CHₐᵣ), 5.68 (1 H, s, CHOH), 4.81 (1 H, d, *J* 12.8, ArCHₐH_{b}), 4.13 (1 H, m, OCHₐH_{b}), 3.83 (1H, OCHₐH_{b}), 3.76 (3H, s, OCH₃), 3.58 (1 H, d, *J* 12.8, ArCHₐH_{b}), 2.77 (1 H, m NCHₐH_{b}), 2.61 (1 H, ddd, *J* 2.5, 5.7, 14.1, NCHₐH_{b}), 2.45 (3H, s, CH₃).

### 2-(3-Methoxy)benzoyl-4-bromobenzoic acid and 2-(3-methoxy)benzoyl-5-bromobenzoic acid (18)

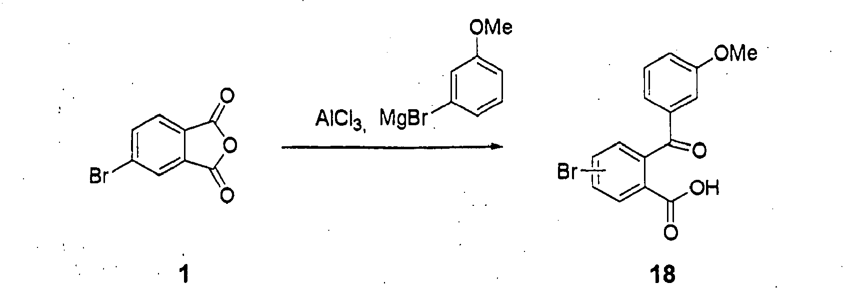

Magnesium (2.45g, 0.1mol) was suspended in anhydrous ether (250ml) under a nitrogen atmosphere at room temperature. 3-Bromoanisole (18.75g, 0.1 mol) and iodine (cat.) were added to form an initial red solution which became colourless after a few minutes. The reaction was allowed to stir overnight and a pale yellow precipitate formed. The precipitate was added, via a dropping funnel, to a stirred solution of 4-bromophthalic anhydride (**1**) (25g, 0.1 mol) in toluene (150ml) and ether (30ml) under a nitrogen atmosphere. The subsequent reflux was maintained for 24 hours, cooled and quenched with saturated aqueous NH₄Cl. Thw aqueous layer was extracted with diethyl ether (3 x 200ml), dried over MgSO₄, filtered and concentrated under reduced pressure to give the desired mixture of regioisomers (30.1 g, 82% yield).

¹H nmr (250MHz, CD₃OD) 8.19 (H, s, CHₐᵣ), 7.92 (H, d, CHₐᵣ), 7.74 (2H, dd, *J* 23.5, 8.7, CHₐᵣ), 7.51 (H. s, CHₐᵣ), 7.36-7.09 (9H, m, CHₐᵣ), 6.95-6.85 (H, m, CHₐᵣ), 6.52-6.40 (H, m, CHₐᵣ), 2.35 (6H, s, OCH₃),

### N-(2-hydroxyethyl)-N-methyl- 2-(3-methoxy)benzoyl-4-bromobenzamide (19a) and N-(2-hydroxyethyl)-N-methyl- 2-(3-methoxy)benzoyl-5-bromobenzamide (19b)

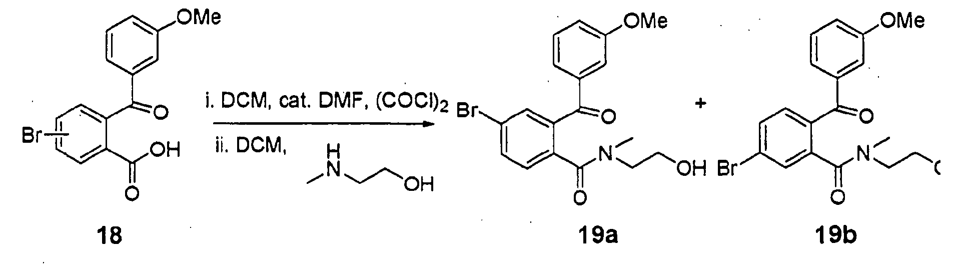

A solution of 2M oxalyl chloride (8.65mL, 0.1mol) in dichloromethane was added dropwise to a suspension of the mixture of isomers **18** (30.0g, 0.09mol) in dichloromethane (200ml) and catalytic *N,N*-dimethylformamide (1 drop) at room temperature under a nitrogen atmosphere. Gas evolution was rapid and as the reaction proceeded the solid dissolved in the dichloromethane. After 2.5 hours the solvent was removed under reduced pressure and the resulting solid co-evaporated with dichloromethane to remove traces of excess oxalyl chloride. The crude acid chloride was dissolved in dichloromethane (200mL) and added dropwise to a solution of N-methylaminoethanol (6mL, 0.1mol.) and triethyamine (14mL) in dichloromethane (200mL) cooled to 0°C in an ice bath. The resulting solution was stirred at room temperature overnight, quenched with saturated aqueous ammonium chloride and separated. The organic layer was washed with water (2 x 400mL), dried over magnesium sulfate and filtered. The solvent was removed under reduced pressure and the crude product purified by chromatography, eluting with hexane : ethyl acetate, (1:1) to yield **19a** (8.88g, 25% yield) and **19b** (6.13g, 18% yield) as viscous oils.

### 19a:

¹H nmr (250MHz, CDCl₃); 7.61-7.56 (3H, m, CHₐᵣ), 7.49-7.44 (H, m, CHₐᵣ), 7.39-7.26 (8H, m, CHₐᵣ), 7.17-7.13 (2H, m, CHₐᵣ), 3.86-3.83 (9H, m, OCH₃, CH₂), 3.61 (4H, t, *J* 5.3, CH₂), 3.09 (3H, s, NCH₃), 2.99 (3H, s, NCH₃), 1.41 (2H, brs, OH).

### N-(2-hydroxyethyl)-N-methyl-4-bromo-2-[1-hydroxy-1-(3-methoxyphenyl)] methylbenzylamine (20a)

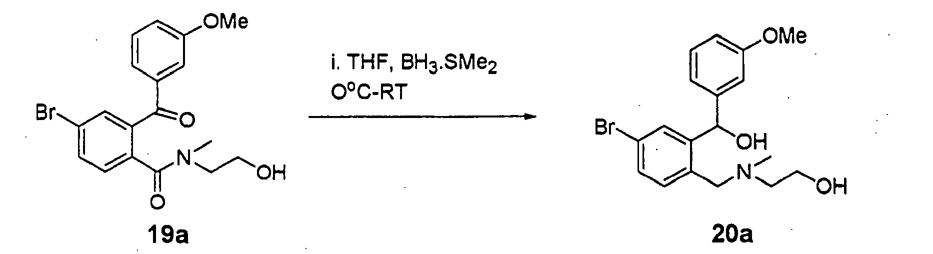

Amide **19a** (700mg, 1.78mmol) was dissolved in tetrahydrofuran (3mL) and cooled to 0°C in an ice bath. A 2M solution of borane dimethylsulfide complex (3.6mL, 7.12mmol, 4.4 equiv.) was added dropwise and the resulting solution stirred at room temperature for 17 hours. The reaction was carefully quenched with 1 M hydrochloric acid solution and the resulting solution stirred overnight. Tetrahydrofuran was removed under reduced pressure and the remaining solution washed with diethyl ether until all by-products had been removed. The aqueous layer was basified with NaOH (2M) and extracted using ethyl acetate (30ml). After washing with NaOH (2x50ml) and water (50ml), the ethyl acetate was dried over MgSO₄, filtered and concentrated under reduced pressure to furnish the desired product **20a** as a viscous oil. Yield 400mg, 59%.

¹H nmr (250MHz, CDCl₃); 7.37-7.31 (2H, m, CHₐᵣ), 7.21 (1H, t, *J* 8.5, CHₐᵣ), 7.05-6.98 (2H, m, CHₐᵣ), 6.85-6.77 (2H, m, CHₐᵣ), 5.75 (1H, s, CHOH), 3.77 (3H, s, OCH₃), 3.65-3.61 (2H, m, OCH₂), 3.29 (2H, dd, *J*, 25.3,13.5, ArCHₐH_{b}N), 2.50 (2H, dd, *J* 10.9, 5.4, NCH₂), 2.02 (3H, s, NCH₃).

### N-(2-hydroxyethyl)-N-methyl-5-bromo-2-[1-hydroxy-1-(4-methoxyphenyl)] methylbenzylamine (20b)

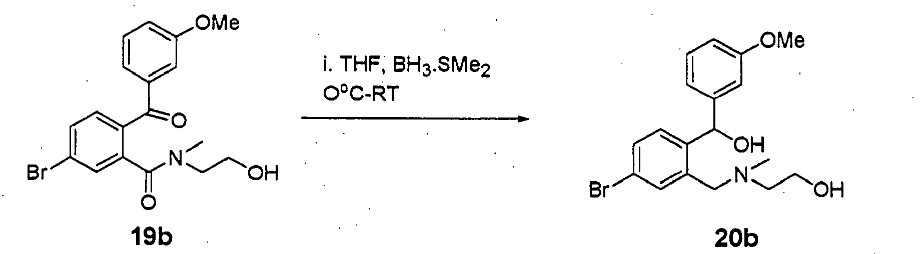

Amide **19b** (550mg, 1.40mmol) was dissolved in tetrahydrofuran (3mL) and cooled to 0°C in an ice bath. A 2M solution of borane dimethylsulfide complex (2.8mL, 5.60mmol, 4.4 equiv.) was added dropwise and the resulting solution stirred at room temperature for 17 hours. The reaction was carefully quenched with 1 M hydrochloric acid solution and the resulting solution stirred overnight. Tetrahydrofuran was removed under reduced pressure and the remaining solution washed with diethyl ether until all by-products had been removed. The aqueous layer was basified with NaOH (2M) and extracted using ethyl acetate (30ml). After washing with NaOH (2x50ml) and water (50ml), the ethyl acetate was dried over MgSO₄, filtered and concentrated under reduced pressure to furnish the desired product **20b** as a viscous oil. Yield 300mg, 56%.

¹H nmr (250MHz, CDCl₃); 7.40-7.35 (3H, m, CHₐᵣ), 7.26-7.20 (2H, m, CHₐᵣ), 7.07 (1H, d, *J* 8.6, CHₐᵣ), 5.80 (1 H, s, CHOH), 3.79 (3H, s, OCH₃) 3.71-3.66 (2H, m, OCH₂), 3.36 (2H, dd, *J*, 19.6, 12.5, ArCH₂N), 2.62-2.49 (2H, m, NCH₂), 2.04 (3H, s, NCH₃).

### 9-Bromo-5-methyl-1-(3-methoxy)phenyl-1,3,4,6-tetrahydro-5H-benz[f]-2,5-oxazocine (21a)

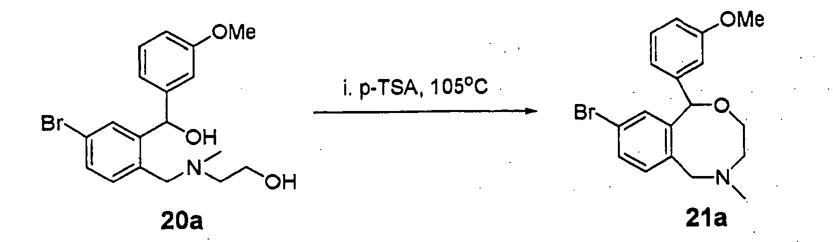

Diol **20a** (400mg, 1.05mmol) was dissolved in toluene (2mL) and *para*toluenesulfonic acid monohydrate (300mg, 1.58mmol, 1.5equiv.) added. The toluene was removed under reduced pressure and the resulting oil heated at 105°C for 4 hours. On cooling the oil was suspended in water (100mL) and basified with 3.75M sodium hydroxide solution. The aqueous layer was extracted with ethyl acetate, dried over magnesium sulfate, filtered and evaporated under reduced pressure to furnish the crude product. Purification by column chorography (10% methanol in ethyl acetate) furnished **21a** as yellow solid. Yield 71mg, 19%.

¹H nmr (250MHz, CDCl₃); 7.39-7.05 (5H, m, CHₐᵣ), 6.84-6.79 (3H, m, CHₐᵣ), 5.66 (1H, s, CH), 4.78 (1H, d, *J* 13.8, ArCHₐH_{b}), 4.22-4.13 (1H, m, OCHₐH_{b}), 3.84-3.83 (1 H, m, OCHₐH_{b}), 3.77 (3H, s, OCH₃), 3.64 (1 H, d, *J* 13.7, ArCHₐH_{b}), 2.82-2.74 (1 H, m NCHₐH_{b}), 2.64-2.57 (1 H, m, CHₐH_{b}), 2.42 (3H, s, CH₃).

### 8-Bromo-5-methyl-1-(3-methoxy)phenyl-1,3,4,6-tetrahydro-5H-benz[f]-2,5-oxazocine (21b)

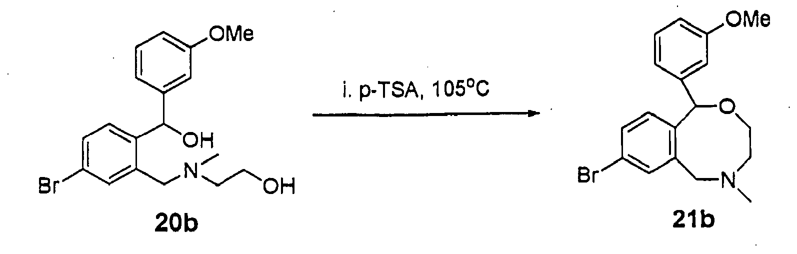

Diol **20b** (300mg, 0.79mmol) was dissolved in toluene (2mL) and paratoluenesulfonic acid monohydrate (228mg, 1.2mmol, 1.5equiv.) added. The toluene was removed under reduced pressure and the resulting oil heated at 105°C for 4 hours. On cooling the oil was dissolved in 3.75M sodium hydroxide solution and extracted with ethyl acetate, dried over magnesium sulfate, filtered and evaporated under reduced pressure to furnish **21b** as a brown oil. Yield 75mg, 26%. No further purification was performed.

¹H nmr (250MHz, CDCl₃); 7.37-719 (3H, m, CHₐᵣ), 6.89-6.78 (4H, m, CHₐᵣ), 5.70((1H, s, CH), 4.75 (1 H, d, *J* 14, ArCHₐH_{b}), 4.20-4.10 (1 H, m, OCHₐH_{b}), 3.86-3.77 (4H, m, ArCHₐH_{b}), OCH3), 3.60 (1 H, d, *J* 14, ArCHₐH_{b}), 2.80(1 H, ddd, *J* 2.1, 8.5, 15.3, NCHₐH_{b}), 2.62 (1 H, ddd, *J* 2.7, 6.4, 15.3, NCHₐH_{b}), 2.44 (3H, s, CH₃).

### 9-Cyano-5-methyl-1 -(3-methoxy)phenyl-1,3,4,6-tetrahydro-5H-benz[f]-2,5-oxazocine (22a)

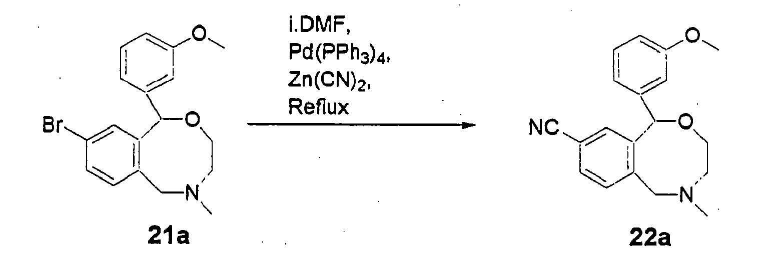

Bromo-nefopam analogue **21a** (503mg, 1.39mmol), Zn(CN)₂ (245mg, 2.09mmol), and Pd(PPh₃)₄ (241mg, 0.21mmol), were dissolved in degassed anhydrous DMF (1 0mL) under a N₂ atmosphere. The mixture was refluxed under N₂ for 24 hours. The mixture was allowed to cool to room temperature, filtered through celite and washed through with DCM (50ml). The filtrate was then quenched with water (10ml) and solvent extracted. The organic extract was dried over MgSO₄, filtered and solvent removed under reduced pressure. The crude product was purified by dry flash chromatography eluting with 5%-15% MeOH in DCM. Fractions containing the product were combined and evaporated under reduced pressure to produce **22a** as pale brown oil. Yield 143mg, 33%.

¹H nmr (250MHz, CDCl₃); 7.51 (1 H, dd, *J* 1.4, 7.8, CHₐᵣ), 7.32-7.23 (4H, m, CHₐᵣ), 6.84-6.81 (3H, m, CHₐᵣ), 5.76 (1 H, s, CHO), 4.85 (1 H, d, *J* 12.8, ArCHₐH_{b}), 4.18-4.15 (1 H, m, OCHₐH_{b}), 3.86-3.81 (1 H, m, OCHₐH_{b}), 3.79 (3H, s, OCH₃), 3.71 (1H, d, *J* 12.8, ArCHₐH_{b}), 2.78 (1H, ddd, *J* 2.3, 8.2, 14.3, NCHₐH_{b}), 2.63 (1H, ddd, *J* 2.8, 5.6, 14.3, NCHₐH_{b}), 2.44 (3H, s, NCH₃).

### 8-Cyano-5-methyl-1-(3-methoxy)phenyl-1,3,4,6-tetrahydro-5H-benz[f]-2,5-oxazocine (22b)

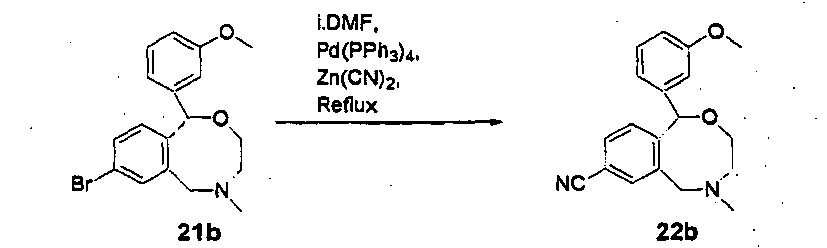

Bromo-nefopam analogue **21b** (223mg, 0.62mmol), Zn(CN)₂ (109mg, 0.93mmol), and Pd(PPh₃)₄ (107mg, 0.09mmol), were dissolved in degassed anhydrous DMF (5mL) under a N₂ atmosphere. The mixture was refluxed under N₂ for 24 hours. The mixture was allowed to cool to room temperature, filtered through celite and washed through with DCM (50ml). The filtrate was then quenched with water (10ml) and solvent extracted. The organic extract was dried over MgSO₄, filtered and solvent removed under reduced pressure. The crude product was purified by dry flash chromatography eluting with 5%-15% MeOH in DCM. Fractions containing the product were combined and evaporated under reduced pressure to produce **22b** as pale brown solid. Yield 94mg, 50%.

¹H nmr (250MHz, CDCl₃); 7.50 (1 H, s, CHₐᵣ), 7.46 (1H, dd, *J* 1.7, 8.1, CHₐᵣ), 7.26-7.21 (1 H, m, CHₐᵣ), 7.12 (1 H, d, *J* 7.9, CHₐᵣ), 6.83-6.80 (3H, m, CHₐᵣ), 5.74 (1 H, s, CHO), 4.94 (1 H, d, *J* 13.0, ArCHₐH_{b}), 4.28-4.19 (1H, m, OCHₐH_{b}), 3.87-3.79 (1H, m, OCHₐH_{b}), 3.77 (3H, s, OCH₃), 3.65 (1H, d, *J* 13.0, ArCHₐH_{b}), 2.75 (1H, ddd, *J* 2.3, 8.3, 14.3, NCHₐH_{b}), 2.63 (1H, ddd, *J* 3.0, 5.4, 14.3, NCHₐH_{b}), 2.45 (3H, s, NCH₃).

### 9-Methoxy-5-methyl-1-(3-methoxy)phenyl-1,3,4,6-tetrahydro-5H-benz[f]-2,5-oxazocine (24a)

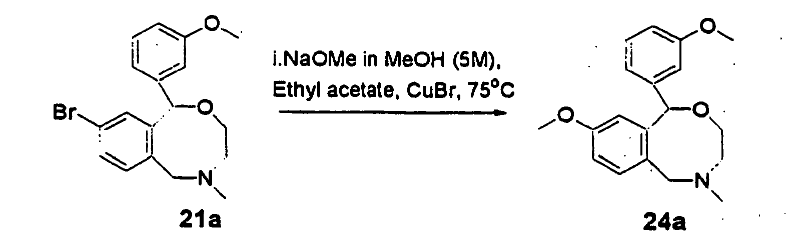

Ethyl acetate (0.1 ml) was added to a stirred 5M NaOMe methanol solution (1ml) under an N₂ atmosphere at room temperature. Bromo-nefopam analogue **21a** (173mg, 0.48mmol) in MeOH (1 ml) was then added followed by CuBr (14mg. 0.10mmol). The mixture was stirred at 75°C overnight, cooled to room temperature and quenched using water (5ml). The organic layer was separated and the aqueous was washed with ethyl acetate (2x10ml). The combined organics were dried over MgSO₄, filtered and concentrated under reduced pressure. The crude product was purified by dry flash chromatography eluting with 10% MeOH in DCM. Fractions containing the product were combined and evaporated under reduced pressure to produce **24a** as an orange oil. Yield 34mg, 22%.

¹H nmr (250MHz, CDCl₃); 7.20 (1 H, d, *J* 8.2, CHₐᵣ), 7.15 (1 H, s, CHₐᵣ), 6.87-6.84 (3H, m, CHₐᵣ), 6.77 (1 H, dd, *J* 2.6, 8.3, CHₐᵣ), 6.54 (1 H, d, *J* 2.6, CHₐᵣ), 5.68 (1 H, s, CHO), 4.75 (1 H, d, *J* 12.8, ArCHₐH_{b}), 4.24-4.15 (1 H, m, OCHₐH_{b}), 3.85 (1 H, ddd, *J* 2.4, 5.3, 12.7, OCHₐH_{b}), 3.76 (3H, s, OCH₃), 3.71 (1H, d, *J* 12.8, ArCHₐH_{b}), 3.70 (3H, s, OCH₃), 2.85 (1H, ddd, *J* 2.1, 8.7, 14.1, NCHₐH_{b}), 2.64 (1H, ddd, *J* 2.7, 5.3, 14.3, NCHₐH_{b}), 2.47 (3H, s, NCH₃).

### 8-Methoxy-5-methyl-1-(3-methoxy)phenyl-1,3,4,6-tetrahydro-5H-benz[f]-2,5-oxazocine (24b)

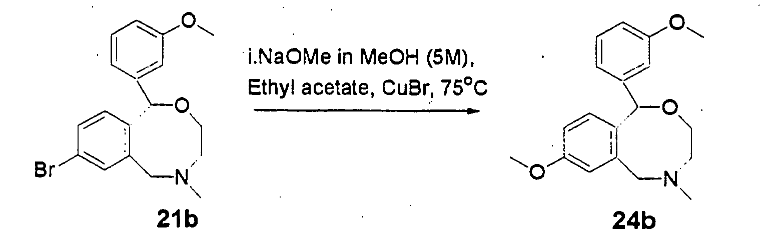

Ethyl acetate (0.1 ml) was added to a stirred 5M NaOMe methanol solution (1ml) under an N₂ atmosphere at room temperature. Bromo-nefopam analogue **21b** (168mg, 0.47mmol) in MeOH (1 ml) was then added followed by CuBr (13mg, 0.09mmol). The mixture was stirred at 75°C overnight, cooled to room temperature and quenched using water (5ml). The organic layer was separated and the aqueous was washed with ethyl acetate (2x10ml). The combined organics were dried over MgSO₄, filtered and concentrated under reduced pressure. The crude product was purified by dry flash chromatography eluting with 10% MeOH in DCM. Fractions containing the product were combined and evaporated under reduced pressure to produce **24b** as an orange oil. Yield 77mg, 50%.

¹H nmr (250MHz, CDCl₃); 7.22 (1H, t, *J* 8.2, CHₐᵣ), 6.92-6.71 (6H, m, CHₐᵣ), 5.78 (1H, s, CHO), 4.60 (1H, d, *J* 12.7, ArCHₐH_{b}), 4.09 (1H, ddd, *J* 2.4, 7.9, 12.7, OCHₐH_{b}), 3.90-3.82 (1H, m, OCHₐH_{b}), 3.80 (3H, s, OCH₃), 3.77 (3H, s, OCH₃), 3.70 (1H, d, *J* 12.8, ArCHₐH_{b}), 2.85 (1H, ddd, *J* 1.9, 7.9, 14.2, NCHₐH_{b}), 2.65 (1H, ddd, *J* 2.5, 6.1, 14.2, NCHₐH_{b}), 2.47 (3H, s, NCH₃).

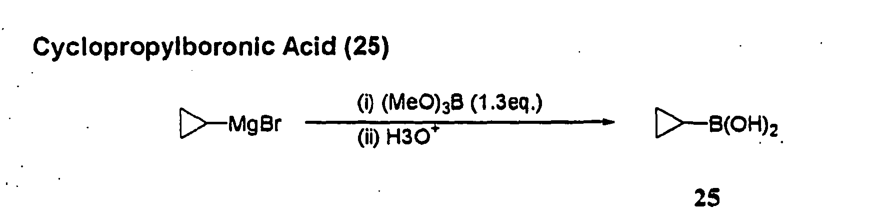

To a stirred solution of trimethylborate (1.69g, 1.81 ml, 16.25mmol) in THF (7ml) at -78°C under a N₂ atmosphere was added, by drop wise addition, cyclopropylmagnesium bromide (0.5M in THF, 25ml, 12.5mmol). A white precipitate formed. After 1 hr the reaction was warmed to room temperature and stirred overnight. The reaction was quenched with HCl aq. (20ml, 2.0N) and the mixture stirred for 1 hour. After extracting with DCM (15ml) and back extracting with H₂O (2x15ml), the aqueous fractions were combined and extracted using. TBME (4x40ml). The combined organic extracts were dried over MgSO₄ and concentrated under reduced pressure to give a white solid. Recrystallisation from-DCM and hexane (twice) furnished a white solid **25**, 297mg, 27% yield.

¹H nmr (250MHz, CDCl₃); 0.56-0.50 (2H, m, CH₂), 0.42-0.40 (2H, m, CH₂), -0.08--0.20 (1 H, m, CH).

### 9-Cyclopropyl-5-methyl-1-(3-methoxy)phenyl-1,3,4,6-tetrahydro-5H-benz[f]-2,5-oxazocine (29a)

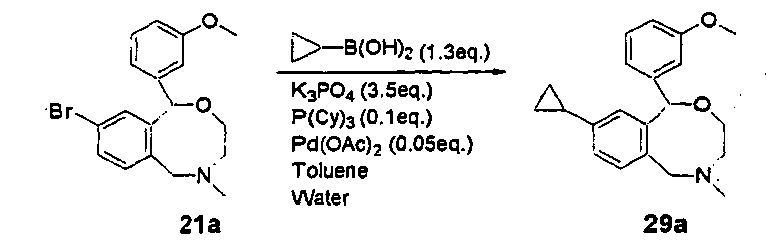

To a stirred solution of bromo-nefopam analogue **21a** (104mg, 0.29mmol), cyclopropylboronic acid 25 (32mg, 0.37mmol), potassium phosphate (214mg, 1.0mmol) and tricyclohexylphosphine (8mg, 0.03mmol) in toluene (5ml) and water (250 µl) under a N₂ atmosphere was added palladium acetate (3mg, 0.01 mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 5% MeOH in DCM. Fractions containing the product were combined and evaporated under reduced pressure to produce **29a** as an orange oil. Yield 65mg, 70%.

¹H nmr (250MHz, CDCl₃); 7.23 (1H, t, *J* 8.1, CHₐᵣ), 7.10 (1H, d, *J* 7.8, CHₐᵣ), 6.88-6.75 (5H, m, CHₐᵣ), 5.70 (1H, s, CHO), 4.75 (1H, d, *J* 12.7, ArCHₐH_{b}), 4.21-4.12 (1H, m, OCHₐH_{b}), 3.84 (1H, ddd, *J* 2.3, 5.7, 12.6, OCHₐH_{b}), 3.77 (3H, s, OCH₃), 3.65 (1H, d, *J* 12.7, ArCHₐH_{b}), 2.82 (1H, ddd, *J* 2.2, 8.3, 14.1, NCHₐH_{b}), 2.62 (1H, ddd, *J* 2.7, 5.7, 14.2, NCHₐH_{b}), 2.45 (3H, s, NCH₃), 1.77 (1H, m, CH), 0.91-0.87 (2H, m, CH₂), 0.62-0.57 (2H, m, CH₂).

### 8-Cyclopropyl-5-methyl-1-(3-methoxy)phenyl-1,3,4,6-tetrahydro-5H-benz[f]-2,5-oxazocine (29b)

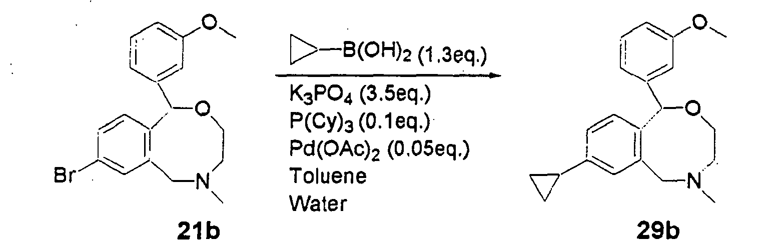

To a stirred solution of bromo-nefopam analogue **21b** (115mg, 0.32mmol), cyclopropyl boronic acid (36mg, 0.42mmol), potassium phosphate (241 mg, 1.13mol) and tricyclohexylphosphine (9mg, 0.03mmol) in toluene (4ml) and water (250 µl) under a N₂ atmosphere was added palladium acetate (4mg, 0.02mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 5% MeOH in DCM. Fractions containing the product were combined and evaporated under reduced pressure to produce **29b** as an orange oil. Yield 77mg, 75%.

¹H nmr (250MHz, CDCl₃); 7.22 (1 H, t, J8.1, CHₐᵣ), 6.94-6.76 (6H, m, CHₐᵣ), 5.74 (1H, s, CHO), 4.69 (1H, d, *J* 12.7, ArCHₐH_{b}), 4.14 (1H, ddd, *J* 2.6, 8.4, 12.6, OCHₐH_{b}), 3.85 (1H, ddd, *J* 2.1, 5.8, 12.7, OCHₐH_{b}), 3.77 (3H, s, OCH₃), 3.68 (1H, d, *J* 12.7, ArCHₐH_{b}), 2.85 (1H, ddd, *J* 2.0, 8.3, 14.2, NCHₐH_{b}), 2.64 (1H, ddd, *J* 2.5, 5.8, 14.3, NCHₐH_{b}), 2.49 (3H, s, NCH₃), 1.86 (1H, m, CH), 0.93 (2H, m, CH₂), 0.68 (2H, m, CH₂).

### 9-Cyclopropyl-5-methyl-1-(4-methoxy)phenyl-1,3,4,6-tetrahydro-5H-benz[f]-2,6-oxazocine (30a)

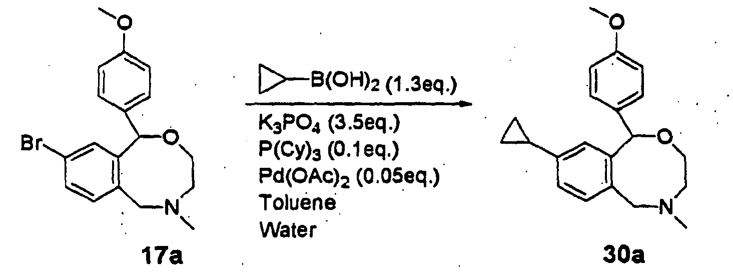

To a stirred solution of bromo-nefopam analogue **17a** (110mg, 0.31 mmol), cyclopropylboronic acid 25 (34mg, 0.40mmol), potassium phosphate (226mg, 1.07mmol) and tricyclohexylphosphine (9mg, 0.03mmol) in toluene (4ml) and water (200 µl) under a N₂ atmosphere was added palladium acetate (4mg, 0.02mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 5% MeOH in DCM. Fractions containing the product were combined and evaporated under reduced pressure to produce **30a** as an orange oil. Yield 70mg, 71%.

¹H nmr (250MHz, CDCl₃); 7.18 (2H, d, *J* 8.5, CHₐᵣ), 7.12 (1H, d, *J* 7.8, CHₐᵣ), 6.88 (1H, d, *J* 1.4, CHₐᵣ), 6.84 (2H, d, J8.7, CHₐᵣ), 6.73 (1H, d, *J* 1.2, CHₐᵣ), 5.68 (1H, s, CHO), 4.80 (1H, d, *J* 12.7, ArCHₐH_{b}), 4.18 (1H, m. OCHₐH_{b}), 3.83 (1H, m, OCHₐH_{b}), 3.77 (3H, s, OCH₃), 3.64 (1H, d, *J* 12.7, ArCHₐH_{b}), 2.81 (1H, ddd, *J* 2.2, 8.5, 14.0, NCHₐH_{b}), 2.60 (1H, ddd, *J* 2.8, 5.5, 14.0, NCHₐH_{b}), 2.46 (3H, s, NCH₃), 1.77 (1H, m, CH), 0.87 (2H, m, CH₂), 0.60 (2H, m, CH₂).

### 8-Cyclopropyl-5-methyl-1-(4-methoxy)phenyl-1,3,4,6-tetrahydro-5H-benz[f]-2,5-oxazocine (30b)

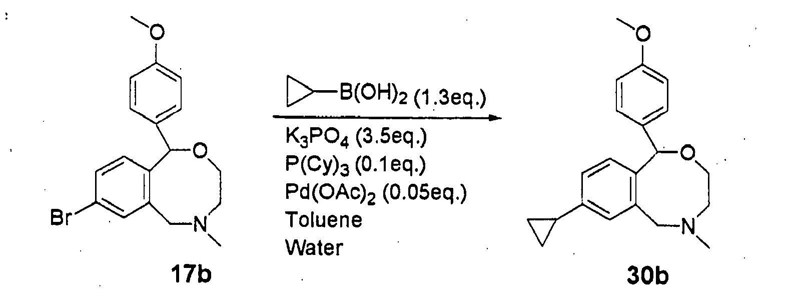

To a stirred solution of bromo-nefopam analogue **17b** (117mg, 0.32mmol), cyclopropyl boronic acid (36mg, 0.42mmol), potassium phosphate (241 mg, 1.13mmol) and tricyclohexylphosphine (9mg, 0.03mmol) in toluene (4ml) and water (200 µl) under a N₂ atmosphere was added palladium acetate (4mg, 0.02mmol). The mixture was heated to 100°C for 3hrs and then cooled to room temperature. Water (10ml) was added and the mixture extracted with ethyl acetate (2x15ml). The combined organic extracts were washed with brine (10ml), dried over MgSO₄ and concentrated under reduced pressure to give a yellow oil. The crude product was purified by dry flash chromatography eluting with 5% MeOH in DCM. Fractions containing the product were combined and evaporated under reduced pressure to produce **30b** as an orange oil. Yield 62mg, 60%.

¹H nmr (250MHz, CDCl₃); 7.17 (2H, d, *J* 8.5, CHₐᵣ), 6.95 (1H, s, CHₐᵣ), 6.84 (4H, m, CHₐᵣ), 5.71 (1H, s, CHO), 4.77 (1H, d, *J* 12.5, ArCHₐH_{b}), 4.17 (1H, m, OCHₐH_{b}), 3.84 (1H, ddd, *J* 2.3, 5.5, 12.6, OCHₐH_{b}), 3.76 (3H, s, OCH₃), 3.66 (1H, d, *J* 12.5, ArCHₐH_{b}), 2.84 (1H, ddd, *J* 2.1, 8.5, 14.0, NCHₐH_{b}), 2.61 (1H, ddd, *J* 2.7, 5.5, 14.1, NCHₐH_{b}), 2.49 (3H, s, NCH₃), 1.85 (1H, m, CH), 0.93 (2H, m, CH₂), 0.67 (2H, m, CH₂).

### 9-Methoxy-5-methyl-1-(4-methoxy)phenyl-1,3,4,6-tetrahydro-5H-benz[f]-2,5-oxazocine (31a)

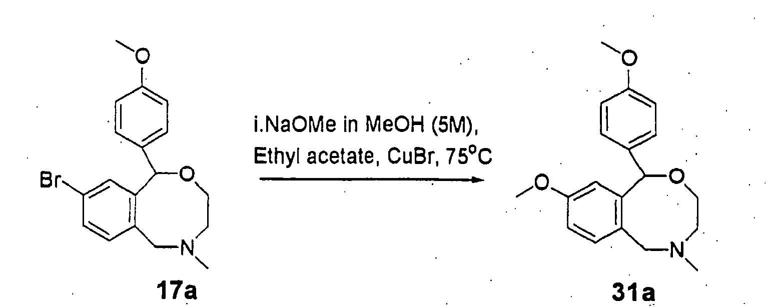

Ethyl acetate (0.1 ml) was added to a stirred 5M NaOMe methanol solution (1ml) under an N₂ atmosphere at room temperature. Bromo-nefopam analogue **17a** (1 04mg, 0.29mmol) in MeOH (1 ml) was then added followed by CuBr (8mg, 0.06mmol). The mixture was stirred at 75°C overnight, cooled to room temperature and quenched using water (5ml). The organic layer was separated and the aqueous was washed with ethyl acetate (2x10ml). The combined organics were dried over MgSO₄, filtered and concentrated under reduced pressure. The crude product was purified by dry flash chromatography eluting with 10% MeOH in DCM. Fractions containing the product were combined and evaporated under reduced pressure to produce **31a** as an orange oil. Yield 67mg, 71%.

¹H nmr (250MHz, CDCl₃); 7.18 (2H, d, *J* 8.5, CHₐᵣ), 7.19 (1H, s, CHₐᵣ), 6.83 (2H, d, *J* 8.6, CHₐᵣ), 6.77 (1H, dd, *J* 2.5, 8.5, CHₐᵣ), 6.52 (1H, d, *J* 2.3, CHₐᵣ), 5.72 (1H, s, CHO), 4.80 (1H, d, *J* 12.8, ArCHₐH_{b}), 4.20 (1H, m, OCHₐH_{b}), 3.84 (1H, ddd, *J* 2.4, 5.1, 12.8, OCHₐH_{b}), 3.77 (3H, s, OCH₃), 3.71 (3H, s, OCH₃), 3.68 (1H, d, *J* 12.8, ArCHₐH_{b}), 2.85 (1H, m, NCHₐH_{b}), 2.61 (1H, m, NCHₐH_{b}); 2.47 (3H, s, NCH₃).

### 8-Methoxy-5-methyl-1-(4-methoxy)phenyl-1,3,4,6-tetrahydro-5H-benz[f]-2,5-oxazocine (31b)

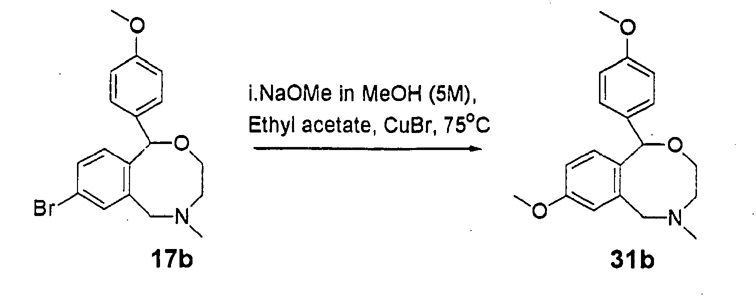

Ethyl acetate (0,1 ml) was added to a stirred 5M NaOMe methanol solution (1ml) under an N₂ atmosphere at room temperature. Bromo-nefopam analogue **17b** (115mg, 0.32mmol) in MeOH (1 ml) was then added followed by CuBr (9mg, 0.06mmol). The mixture was stirred at 75°C overnight, cooled to room temperature and quenched using water (5ml). The organic layer was separated and the aqueous was washed with ethyl acetate (2x10ml). The combined organics were dried over MgSO₄, filtered and concentrated under reduced pressure. The crude product was purified by dry flash chromatography eluting with 10% MeOH in DCM. Fractions containing the product were combined and evaporated under reduced pressure to produce **31b** as an orange oil. Yield 39mg, 37%.

¹H nmr (250MHz, CDCl₃); 7.18 (2H, d, *J* 8.6, CHₐᵣ), 6.91 (1H, s, CHₐᵣ), 6.84 (2H, d, *J* 8.5, CHₐᵣ), 6.76 (1H, s, CHₐᵣ), 6.73 (1H, d, *J* 2.6, CHₐᵣ), 5.73 (1H, s, CHO), 4.71 (1H, d, *J* 12.7, ArCHₐH_{b}), 4.13 (1H, m, OCHₐH_{b}), 3.86 (1H, m, OCHₐH_{b}), 3.80 (3H, s, OCH₃), 3.77 (3H, s, OCH₃), 3.68 (1H, d, *J* 12.7, ArCHₐH_{b}), 2.86 (1H, m, NCHₐH_{b}), 2.64 (1H, ddd, *J* 2.5, 5.7, 14.1 NCHₐH_{b}). 2.48 (3H, s, NCH₃).

### 9-Cyano-5-methyl-1-(4-methoxy)phenyl-1,3,4,6-tetrahydro-5H-benz[f]-2,5-oxazocine (32a)

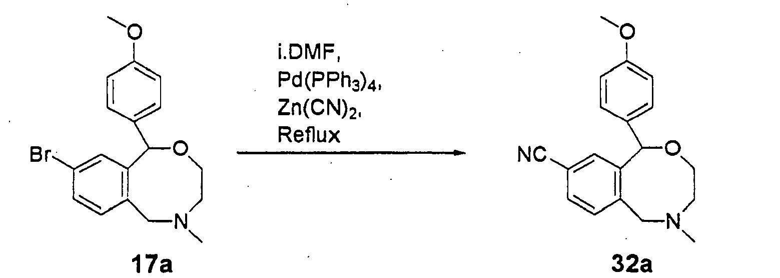

Bromo-nefopam analogue **17a** (133mg, 0.37mmol), Zn(CN)₂ (65mg, 0.55mmol), and Pd(PPh₃)₄ (63mg, 0.06mmol), were dissolved in degassed anhydrous DMF (4mL) under a N₂ atmosphere. The mixture was refluxed under N₂ for 24 hours. The mixture was allowed to cool to room temperature, filtered through celite and washed through with DCM (50ml). The filtrate was then quenched with water (10ml) and solvent extracted. The organic extract was dried over MgSO₄, filtered and solvent removed under reduced pressure. The crude product was purified by dry flash chromatography eluting with 5%-15% MeOH in DCM. Fractions containing the product were combined and evaporated under reduced pressure to produce **32a** as pale brown oil. Yield 32mg, 26%.

¹H nmr (250MHz, CDCl₃); 7.50 (1H, d, *J* 7.6, CHₐᵣ), 7.31 (2H, d, *J* 9.0, CHₐᵣ), 7.15 (2H, d, *J* 8.2, CHₐᵣ), 6.86 (2H, d, *J* 8.2, CHₐᵣ), 5.73 (1H, s, CHO), 4.91 (1H, d, *J* 12.8, ArCHₐH_{b}), 4.20 (1H, m, OCHₐH_{b}), 3.82 (4H, brm, OCHₐH_{b}, OCH₃), 3.68 (1H, d, *J* 12.8, ArCHₐH_{b}), 2.76 (1H, m, NCHₐH_{b}), 2.59 (1H, m, NCHₐH_{b}), 2.44 (3H, s, NCH₃).

### Biological Assays:

The assay was carried out according to the method described in, PEROVIC, S. and MULLER, W.E.G. (1995), Pharmacological profile of hypericum extract: effect on serotonin uptake by postsynaptic receptors, Arzneim-Forsch. Drug Res., 45: 1145.

### Assay for Inhibition of Noradrenaline Reuptake Activity:

The synaptosomes (100 µg) are incubated for 20 min at 37°C with 0.1 µCi [³H]norepinephrine in the absence (control) or presence of the test compound or the reference compound in a buffer containing 118 mM NaCl, 5 mM KCl, 2.5 mM MgSO₄, 1.2 mM NaH₂PO₄, 25 mM NaHCO₃, 11 mM glucose, 10 µM EGTA and 50 µM ascorbic acid (pH 7.4). Basal control activity is determined by incubating the same mixture for 20 min at 0°C in the presence of 10 µM protriptyline to block the uptake. Following incubation, the samples are filtered rapidly under vacuum through glass fiber filters (GF/B, Packard) and rinsed twice with ice-cold incubation buffer using a 96-sample cell harvester (Unifilter, Packard) to eliminate free [³H]norepinephrine. The filters are dried and the retained radioactivity is measured in a scintillation counter (Topcount, Packard) using a scintillation cocktail (Microscint 0, Packard).

The results are expressed as a percent inhibition of the control uptake of [³H]norepinephrine. The standard inhibitory reference compound is protriptyline, which is tested in each experiment at several concentrations to obtain an inhibition curve from which its IC₅₀ value is calculated.

### Assay for Inhibition of Serotonin Reuptake Activity:

The synaptosomes (100 µg) are incubated for 15 min at 37°C with 0.1 µCi [³H]serotonin in the absence (control) or presence of the test compound or the reference compound in a buffer containing 118 mM NaCl, 5 mM KCl, 2.5 mM MgSO₄, 1.2 mM NaH₂PO₄, 25 mM NaHCO₃, 11 mM glucose, 10 µM EGTA and 50 µM ascorbic acid (pH 7.4). Basal control activity is determined by incubating the same mixture for 15 min at 4°C in the presence of 10 µM imipramine to block the uptake. Following incubation, the samples are filtered rapidly under vacuum through glass fiber filters (GF/B, Packard) and rinsed twice with ice-cold incubation buffer using a 96-sample cell harvester (Unifilter, Packard) to eliminate free [³H]serotonin. The filters are dried and the retained radioactivity is measured in a scintillation counter (Topcount, Packard) using a scintillation cocktail (Microscint 0, Packard).

The results are expressed as a percent inhibition of the control uptake of [³H]serotonin. The standard inhibitory reference compound is imipramine, which is tested in each experiment at several concentrations to obtain an inhibition curve from which its IC₅₀ value is calculated.

## Claims

1. A compound of the general formula (1): 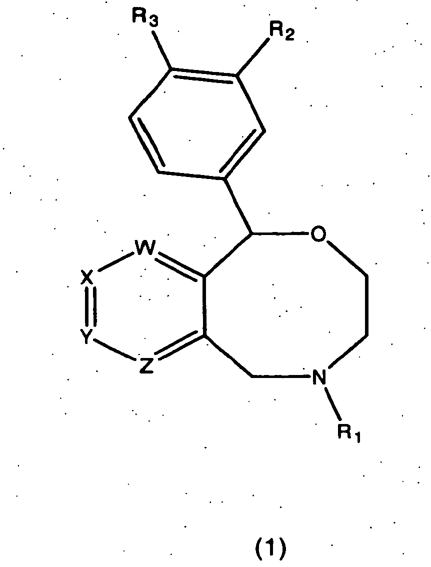 wherein
R₁ is H, C₁-C₆ alkyl optionally substituted with F, C₃-C₆ cycloalkyl or C₂-C₆ alkenyl;
either R₂ and R₃ are the same or different and are H, halogen, CN, CF₃, C₁-C₆ alkyl or OR₁, or R₂ and R₃ form a five or six-membered ring which may be carbocyclic, heterocyclic (containing 1-2 heteroatoms selected from O, N and S), aromatic or heteroaromatic (containing 1-2 heteroatoms selected from O and N);
one of W, X, Y and Z is N or CR₄ and the others are each CH;
R₄ is halogen, CF₃, CN, OR₇, SO₂N(R₆)₂, CORE, CO₂R₆, CON(R₆)₂, NR₁COR₅, NR₁SO₂R₅, NR₁CO₂R₅, NR₁CON(R₆)₂, OC₁-C₆ alkyl optionally substituted with R₄, C₁-C₆ alkyl optionally substituted with R₄, C₃-C₅ cycloalkyl optionally substituted with R₄, C₂-C₆ alkenyl optionally substituted with R₄, C₂-C₆ alkynyl optionally substituted with R₄, aryl optionally substituted with R₄, or a five or six-membered aromatic heterocycle containing 1-4 heteroatoms selected from N and O, linked either through carbon or nitrogen;
R₅ is C₁-C₆ alkyl, C₂-C₆ alkenyl, C₂-C₆ alkynyl, C₃-C₆ cycloalkyl, aryl or heteroaryl;
each R₆ (which may be the same or different) is H, C₁-C₆ alkyl, C₂-C₆ alkenyl, C₂-C₆ alkynyl, C₃-C₆ cycloalkyl, aryl or heteroaryl; and
R₇ is aryl or heteroaryl;
wherein aryl is an optionally substituted phenyl or naphthyl group;
carbocyclic is a saturated alicyclic moiety having 5 or 6 C atoms;
heterocyclic is a saturated heterocyclic moiety having 5 or 6 atoms including one or more of N, O and S; and
heteroaromatic is aromatic of 5 or 6 atoms of which at least one is N, O or S;
provided that R₂ and R₃ are not both hydrogen;
or a pharmaceutically acceptable salt thereof.

2. A compound of claim. 1, wherein R₄ is present and is CN, OR₇, SO₂N(R₆)₂, COR₆, CO₂R₆, CON(R₆)₂, NR₁COR₅, NR₁SO₂R₅, NR₁CON(R₆)₂, OC₁-C₆ alkyl substituted with R₄, C₁-C₆ alkyl substituted with R₄, C₃-C₆ cycloalkyl optionally substituted with R₄, C₂-C₆ alkenyl optionally substituted with R₄, C₂-C₆ alkynyl optionally substituted with R₄, aryl optionally substituted with R₄, or a five or six-membered aromatic heterocycle containing 1-4 heteroatoms selected from N and O, linked either through carbon or nitrogen.

3. A compound of claim 2, wherein R₄ is CN, CON(R₆)₂, optionally substituted cycloalkyl or aryl, or a five or six-membered aromatic heterocycle.

4. A compound of any of claims 1 to 3, wherein R₂ is halogen, CN, CF₃, C₁-C₆ alkyl or OR₁.

5. A compound of any of claims 1 to 3, wherein R₂ and R₃ form a ring.

6. A compound of claim 4, wherein R₂ or R₃ is OR₁.

7. A compound of claim 1, selected from
9-methoxy-5-methyl-1-(3-methoxy)phenyl-1,3,4,6-tetrahydro-5H-benz[f]-2,5-oxazocine (24a)
8-methoxy-5-methyl-1-(3-methoxy)phenyl-1,3,4,6-tetrahydro-5H-benz[f]-2,5-oxazocine (24b)
9-methoxy-5-methyl-1-(4-methoxy)phenyl-1,3,4,6-tetrahydro-5H-benz[f]-2,5-oxazocine (31a) and
8-methoxy-5-methyl-1-(4-methoxy)phenyl-1,3,4,6-tetrahydro-5H-benz[f]-2,5-oxazocine (31b).

8. A compound of claim 1, selected from
9-cyano-5-methyl-1-(3-methoxy)phenyl-1,3,4,6-tetrahydro-5H-benz[f]-2,5-oxazocine (22a) and
8-cyano-5-methyl-1-(3-methoxy)pheny)-1,3,4,6-tetrahydro-5H-benz[f]-2,5-oxazocine (22b).

9. A compound of claim 1, selected from
9-cyclopropyl-5-methyl-1-(3-methoxy)phenyl-1,3,4,6-tetrahydro-5H-benz[f]-2,5-oxazocine (29a)
8-cyclopropyl-5-methyl-1-(3-methoxy)phenyl-1,3,4,6-tetrahydro-5H-benz[f]-2,5-bxazocine (29b)
9-cyclopropyl-5-methyl-1-(4-methoxy)phenyl-1,3,4,6-tetrahydro-5H-benz[f]-2,5-oxazocine (30a)
8-cyclopropyl-5-methyl-1 -(4-methoxy)phenyl-1,3,4,6-tetrahydro-5H-benz[f]-2,5-oxazocine (30b) and
9-cyano-5-methyl-1-(4-methoxy)phenyl-1,3,4,6-tetrahydro-5H-benz[f]-2,5-oxazocine (32a)
9-cyano-5-methyl-1-phenyl-1,3,4,6-tetrahydro-5H-benz[f]-2,5-oxazocine (6a)
8-cyano-5-methyl-1-phenyl-1,3,4,6-tetrahydro-5H-benz[f]-2,5-oxazocine (6b)
5-methyl-1-phenyl-1,3,4,6-tetrahydro-5H-benz[f]-2,5-oxazocine-9-carboxamide (7a)
5-methyl-1-phenyl-1,3,4,6-tetrahydro-5H-benz[f]-2,5-oxazocine-8-carboxamide (7b)
*N*-(1,1,1-trimethylmethoxycarbonyl)-5-methyl-1-phenyl-1,3,4,6-tetrahydro-5H-benz[f]-2,5-oxazocine-9-methylamine (8a)
*N*-(1,1,1-trimethylmethoxycarbonyl)-5-methyl-1-phenyl-1,3,4,6-tetrahydro-5H-benz[f]-2,5-oxazocine-8-methylamine (8b)
5-methyl-1,9-diphenyl-1,3,4,6-tetrahydro-5H-benz[f]-2,5-oxazocine (9a)
5-methyl-1,8-diphenyl-1,3,4,6-tetrahydro-5H-benz[f]-2,5-oxazocine (9b)
9-(3,5-dimethylisoxazol-4-yl)-5-methyl-1-phenyl-1,3,4,6-tetrahydro-5H-benz[f]-2,5-oxazocine (10a)
8-(3,5-dimethylisoxazol-4-yl)-5-methyl-1-phenyl-1,3,4,6-tetrahydro-5H-benz[f]-2,5-oxazocine (10b)
2-(5-methyl-1-phenyl-1,3,4,6-tetrahydro-5H-benz[f]-2,5-oxazocine-9-ethenyl)carboxamide (11 a)
2-(5-methyl-1-phenyl-1,3,4,6-tetrahydro-5H-benz[f]-2,5-oxazocine-8-ethenyl)carboxamide (11 b)
2-(5-methyl-1-phenyl-1,3,4,6-tetrahydro-5H-benz[f]-2,5-oxazocine-9-ethyl)carboxamide (12a)
2-(5-methyl-1-phenyl-1,3,4,6-tetrahydro-5H-benz[f]-2,5-oxazocine-8-ethyl)carboxamide (12b)
5-methyl-1-phenyl-1,3,4,6-tetrahydro-5H-benz[f]-2,5-oxazocine-9-methylamine (13a)
5-methyl-1-phenyl-1,3,4,6-tetrahydro-5H-benz[f]-2,5-oxazocine-8-methylamine (13b) and
*N*-(acetyl)-5-methyl-1-phenyl-1,3,4,5-tetrahydro-5H-benz[f]-2,5-oxazocine-9-methylamine (27a).

10. A compound of claim 1, selected from
9-bromo-5-methyl-1-(4-methoxy)phenyl-1,3,4,6-tetrahydro-5H-benz[f]-2,5-oxazocine (17a)
8-bromo-5-methyl-1-(4-methoxy)phenyl-1,3,4,6-tetrahydro-5H-benz[f]-2,5-oxazocine (17b)
9-bromo-5-methyl-1-(3-methoxy)phenyl-1,3,4,6-tetrahydro-5H-benz[f]-2,5-oxazocine (21a) and
8-bromo-5-methyl-1-(3-methoxy)phenyl-1,3,4,6-tetrahydro-5H-benz[t]-2,5-oxazocine (21b).

11. A pharmaceutical composition for use in therapy, comprising a compound of any of claims 1 to 10, and a pharmaceutically acceptable diluent or carrier.

12. Use of a compound of any of claims 1 to 10, for the manufacture of a human or veterinary medicament for the treatment or prevention of a condition selected from depression, post-traumatic stress disorders, attention-deficit disorders, obsessive compulsive disorders, pre-menstrual syndrome, substance abuse, micturition disorders and sexual dysfunction.

13. Use of a compound of any of claims. 1 to 10, for the manufacture of a human or veterinary medicament for the treatment of acute, chronic or neuropathic pain.

14. Use according to claim 13, wherein the pain is associated with cancer, surgery, arthritis, dental surgery, painful neuropathies, trauma, musculoskeletal injury or disease or a visceral disease, or migraine headache.

15. Use according to claim 13 or claim 14, wherein the subject is also treated with an opiate.

16. Use according to claim 13 or claim 14, wherein the subject is also treated with an analgesia inducer selected from acetaminophen, a non-steroidal anti-inflammatory drug, a narcotic analgesic, a local anesthetic, an NMDA antagonist, a neuroleptic agent, an anti-convulsant, an anti-spasmodic, an antidepressant or a muscle relaxant.

17. Use of a compound of any of claims 1 to 10, for the manufacture of a human or veterinary medicament for the treatment or prevention of emesis.

18. Use according to claim 17, wherein the emesis is acute, delayed, postoperative, last-phase, or anticipatory emesis.

19. Use according to claim 17, wherein the emesis is induced by chemotherapy, radiation, toxins, pregnancy, vestibular disorder, motion, postoperative sickness, surgery, gastrointestinal obstruction, reduced gastrointestinal motility, visceral pain, migraine or opioid analgesics.

## Patentansprüche

1. Verbindung der allgemeinen Formel (1): 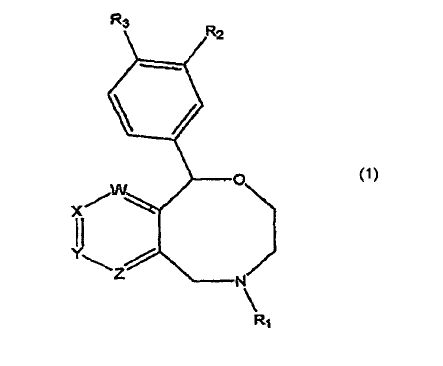 in der
R₁ für H, gegebenenfalls mit F substituiertes (C₁-C₆)-Alkyl, (C₃-C₆)-Cycloalkyl oder (C₂-C₆)-Alkenyl steht;
entweder R₂ und R₃ gleich oder verschieden sind und für H, Halogen, CN, CF₃, (C₁-C₆)-Alkyl oder OR₁ stehen oder R₂ und R₃ einen fünf- oder sechsgliedrigen Ring bilden, der carbocyclisch, heterocyclisch (enthaltend 1-2 Heteroatome, die aus O, N und S ausgewählt sind), aromatisch oder heteroaromatisch (enthaltend 1-2 Heteroatome, die aus O und N ausgewählt sind) sein kann;
eines von W, X, Y und Z für N oder CR₄ steht und die anderen jeweils CH sind;
R₄ Halogen, CF₃, CN, OR₇, SO₂N(R₆)₂, COR₆, CO₂R₆, CON(R₆)₂, NR₁COR₅, NR₁SO₂R₅, NR₁CO₂R₅, NR₁CON(R₆)₂, gegebenenfalls mit R₄ substituiertes O-(C₁-C₆)-Alkyl, gegebenenfalls mit R₄ substituiertes (C₁-C₆)-Alkyl, gegebenenfalls mit R₄ substituiertes (C₃-C₆)-Cycloalkyl, gegebenenfalls mit R₄ substituiertes (C₂-C₆)-Alkenyl, gegebenenfalls mit R₄ substituiertes (C₂-C₆)-Alkinyl, gegebenenfalls mit R₄ substituiertes Aryl, oder ein fünf- oder sechsgliedriger aromatischer Heterocyclus ist, der 1-4 Heteroatome enthält, die aus N und O ausgewählt sind, und entweder durch Kohlenstoff oder durch Stickstoff verknüpft ist;
R₅ für (C₁-C₆)-Alkyl, (C₂-C₆)-Alkenyl, (C₂-C₆)-Alkinyl, (C₃-C₆)-Cycloalkyl, Aryl oder Heteroaryl steht;
jedes R₆ (die gleich oder verschieden sein können) für H, (C₁-C₆)-Alkyl, (C₂-C₆)-Alkenyl, (C₂-C₆)-Alkinyl, (C₃-C₆)-Cycloalkyl, Aryl oder Heteroaryl steht; und
R₇ Aryl oder Heteroarly ist;
wobei Aryl eine gegebenenfalls substituierte Phenyl- oder Naphthylgruppe ist;
carbocyclisch eine gesättigte alicyclische Einheit mit 5 oder 6 C-Atomen ist; heterocyclisch eine gesättigte heterocyclische Einheit mit 5 oder 6 Atomen ist, die eines oder mehrere von N, O und S einschließen; und
heteroaromatisch aromatisch mit 5 oder 6 Atomen ist, von denen mindestens eines N, O oder S ist;
mit der Maßgabe, dass R₂ und R₃ nicht beide Wasserstoff sind;
oder ein pharmazeutisch annehmbares Salz derselben.

2. Verbindung nach Anspruch 1, in der R₄ vorliegt und für CN, OR₇, SO₂N(R₆)₂, COR₆, CO₂R₆, CON(R₆)₂, NR₁COR₅, NR₁SO₂R₅, NR₁CON(R₆)₂, mit R₄ substituiertes O-(C₁-C₆)-Alkyl, mit R₄ substituiertes (C₁-C₆)-Alkyl, gegebenenfalls mit R₄ substituiertes (C₃-C₆)-Cycloalkyl, gegebenenfalls mit R₄ substituiertes (C₂-C₆)-Alkenyl, gegebenenfalls mit R₄ substituiertes (C₂-C₆)-Alkinyl, gegebenenfalls mit R₄ substituiertes Aryl oder ein fünf- oder sechsgliedriger aromatischen Heterocyclus steht, der 1-4 Heteroatome enthält, die aus N und O ausgewählt sind, und entweder durch Kohlenstoff oder durch Stickstoff verknüpft ist.

3. Verbindung nach Anspruch 2, in der R₄ für CN, CON(R₆)₂, gegebenenfalls substituiertes Cycloalkyl oder Aryl oder für einen fünf- oder sechsgliedrigen aromatischen Heterocyclus steht.

4. Verbindung nach irgendeinem der Ansprüche 1 bis 3, in der R₂ Halogen, CN, CF₃, (C₁-C₆)-Alkyl oder OR₁ ist.

5. Verbindung nach irgendeinem der Ansprüche 1 bis 3, in der R₂ und R₃ einen Ring bilden.

6. Verbindung nach Anspruch 4, in der R₂ oder R₃ für OR₁ steht.

7. Verbindung nach Anspruch 1, ausgewählt aus
9-Methoxy-5-methyl-1-(3-methoxy)phenyl-1,3,4,6-tetrahydro-5H-benz[f]-2,5-oxazocin (24a)
8-Methoxy-5-methyl-1-(3-methoxy)phenyl-1,3,4,6-tetrahydro-5H-benz[f]-2,5-oxazocin (24b)
9-Methoxy-5-methyl-1-(4-methoxy)phenyl-1,3,4,6-tetrahydro-5H-benz[f]-2,5-oxazocin (31 a) und
8-Methoxy-5-methyl-1-(4-methoxy)phenyl-1,3,4,6-tetrahydro-5H-benz[f]-2,5-oxazocin (31 b).

8. Verbindung nach Anspruch 1, ausgewählt aus
9-Cyano-5-methyl-1-(3-methoxy)phenyl-1,3,4,6-tetrahydro-5H-benz[f]-2,5-oxazocin (22a) und
8-Cyano-5-methyl-1-(3-methoxy)phenyl-1,3,4,6-tetrahydro-5H-benz[f]-2,5-oxazocin (22b).

9. Verbindung nach Anspruch 1, ausgewählt aus
9-Cyclopropyl-5-methyl-1-(3-methoxy)phenyl-1,3,4,6-tetrahydro-5H-benz[f]-2,5-oxazocin (29a)
8-Cyclopropyl-5-methyl-1-(3-methoxy)phenyl-1,3,4,6-tetrahydro-5H-benz[f]-2,5-oxazocin (29b)
9-Cyclopropyl-5-methyl-1-(4-methoxy)phenyl-1,3,4,6-tetrahydro-5H-benz[f]-2,5-oxazocin (30a)
8-Cyclopropyl-5-methyl-1-(4-methoxy)phenyl-1,3,4,6-tetrahydro-5H-benz[f]-2,5-oxazocin (30b) und
9-Cyano-5-methyl-1-(4-methoxy)phenyl-1,3,4,6-tetrahydro-5H-benz[f]-2,5-oxazocin (32a),
9-Cyano-5-methyl-1-phenyl-1,3,4,6-tetrahydro-5H-benz[f]-2,5-oxazocin (6a)
8-Cyano-5-methyl-1-phenyl-1,3,4,6-tetrahydro-5H-benz[f]-2,5-oxazocin (6b)
5-Methyl-1-phenyl-1,3,4,6-tetrahydro-5H-benz[f]-2,5-oxazocin-9-carboxamid (7a)
5-Methyl-1-phenyl-1,3,4,6-tetrahydro-5H-benz[f]-2,5-oxazocin-8-carboxamid (7b)
*N*-(1,1,1-Trimethylmethoxycarbonyl)-5-methyl-1-phenyl-1,3,4,6-tetrahydro-5H-benz[f]-2,5-oxazocin-9-methylamin (8a)
*N*-(1,1,1-Trimethylmethoxycarbonyl)-5-methyl-1-phenyl-1,3,4,6-tetrahydro-5H-benz[f]-2,5-oxazocin-8-methylamin (8b)
5-Methyl-1,9-diphenyl-1,3,4,6-tetrahydro-5H-benz[f]-2,5-oxazocin (9a)
5-Methyl-1,8-diphenyl-1,3,4,6-tetrahydro-5H-benz[f]-2,5-oxazocin (9b)
9-(3,5-Dimethylisoxazol-4-yl)-5-methyl-1-phenyl-1,3,4,6-tetrahydro-5H-benz[f]-2,5-oxazocin (10a)
8-(3,5-Dimethylisoxazol-4-yl)-5-methyl-1-phenyl-1,3,4,6-tetrahydro-5H-benz[f]-2,5-oxazocin (10b)
2-(5-Methyl-1-phenyl-1,3,4,6-tetrahydro-5H-benz[f]-2,5-oxazocin-9-ethenyl)-carboxamid (11a)
2-(5-Methyl-1-phenyl-1,3,4,6-tetrahydro-5H-benz[f]-2,5-oxazocin-8-ethenyl)-carboxamid (11 b)
2-(5-Methyl-1-phenyl-1,3,4,6-tetrahydro-5H-benz[f]-2,5-oxazocin-9-ethyl)-carboxamid (12a)
2-(5-Methyl-1-phenyl-1,3,4,6-tetrahydro-5H-benz[f]-2,5-oxazocin-8-ethyl)-carboxamid (12b)
5-Methyl-1-phenyl-1,3,4,6-tetrahydro-5H-benz[f]-2,5-oxazocin-9-methylamin (13a)
5-Methyl-1-phenyl-1,3,4,6-tetrahydro-5H-benz[f]-2,5-oxazocin-8-methylamin (13b) und
*N*-(Acetyl)-5-methyl-1-phenyl-1,3,4,6-tetrahydro-5H-benz[f]-2,5-oxazocin-9-methylamin (27a).

10. Verbindung nach Anspruch 1, ausgewählt aus
9-Brom-5-methyl-1-(4-methoxy)phenyl-1,3,4,6-tetrahydro-5H-benz[f]-2,5-oxazocin (17a)
8-Brom-5-methyl-1-(4-methoxy)phenyl-1,3,4,6-tetrahydro-5H-benz[f]-2,5-oxazocin (17b)
9-Brom-5-methyl-1-(3-methoxy)phenyl-1,3,4,6-tetrahydro-5H-benz[f]-2,5-oxazocin (21a) und
8-Brom-5-methyl-1-(3-methoxy)phenyl-1,3,4,6-tetrahydro-5H-benz[f]-2,5-oxazocin (21 b).

11. Pharmazeutische Zusammensetzung zur Verwendung in der Therapie, umfassend eine Verbindung nach irgendeinem der Ansprüche 1 bis 10 und ein pharmazeutisch annehmbares Verdünnungsmittel oder einen pharmazeutisch annehmbaren Träger.

12. Verwendung einer Verbindung nach irgendeinem der Ansprüche 1 bis 10 für die Herstellung eines humanen oder veterinärmedizinischen Medikaments für die Behandlung oder Verhütung eines Zustands, der ausgewählt ist aus Depression, posttraumatischen Stressstörungen, Aufmerksamkeits-Defizit-Störungen, obsessiven Zwangsstörungen, prämenstruellem Syndrom, Substanzabusus, Miktionsstörungen und sexueller Dysfunktion.

13. Verwendung einer Verbindung nach irgendeinem der Ansprüche 1 bis 10 für die Herstellung eines humanen oder veterinärmedizinischen Medikaments für die Behandlung von akutem, chronischem oder neuropathischem Schmerz.

14. Verwendung nach Anspruch 13, bei der der Schmerz mit Krebs, einer Operation, Arthritis, einer Zahnoperation, schmerzenden Neuropathien, Trauma, mit Muskulatur und Skelett in Beziehung stehender Verletzung oder Krankheit oder einer Eingeweideerkrankung oder Migränekopfschmerzen verbunden ist.

15. Verwendung nach Anspruch 13 oder Anspruch 14, bei der der Patient auch mit einem Opiat behandelt wird.

16. Verwendung nach Anspruch 13 oder Anspruch 14, bei der der Patient auch mit einem Analgesie induzierenden Mittel behandelt wird, das ausgewählt ist aus Acetaminophen, einem nicht-steroidalen entzündungshemmenden Medikament, einem narkotischen Analgetikum, einem Lokalanästhetikum, einem NMDA-Antagonisten, einem neuroleptischen Mittel, einem Antikonvulsivum, einem Antispasmodikum, einem Antidepessivum oder einem Muskelrelaxans.

17. Verwendung einer Verbindung nach irgendeinem der Ansprüche 1 bis 10 für die Herstellung eines humanen oder veterinärmedizinischen Medikaments für die Behandlung oder Verhütung von Erbrechen.

18. Verwendung nach Anspruch 17, bei der das Erbrechen ein akutes, verzögertes, postoperatives, Endphasen- oder vorgreifendes Erbrechen ist.

19. Verwendung nach Anspruch 17, bei der das Erbrechen durch Chemotherapie, Strahlung, Toxine, Schwangerschaft, Vestibularstörung, Bewegung, postoperative Übelkeit, Operation, Magen-Darm-Verschluss, verringerte gastrointestinale Motilität, Eingeweideschmerzen, Migräne oder Opioid-Analgetika induziert ist.

## Revendications

1. Composé de formule générale (1): 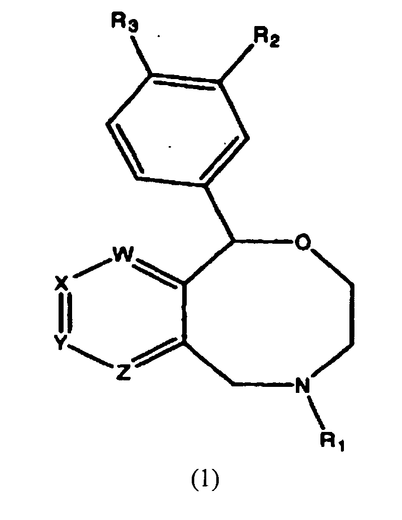 dans laquelle
R₁ est H, alkyle en C₁-C₆ éventuellement substitué par F, cycloalkyle en C₃-C₆ ou alcényle en C₂-C₆;
soit R₂ et R₃ sont identiques ou différents et sont H, halogène, CN, CF₃, alkyle en C₁-C₆ ou OR₁, soit R₂ et R₃ forment un cycle de 5 ou 6 chaînons qui peut être carbocyclique, hétérocyclique (contenant 1-2 hétéroatomes choisis parmi O, N et S), aromatique ou hétéroaromatique (contenant 1-2 hétéroatomes choisis parmi O et N);
l'un des W, X, Y et Z est N ou CR₄ et les autres sont chacun CH;
R₄ est halogène, CF₃, CN, OR₇, SO₂N(R₆)₂, COR₆, CO₂R₆, CON(R₆)₂, NR₁COR₅, NR₁SO₂R₅, NR₁CO₂R₅, NR₁CON(R₆)₂, O-alkyle en C₁-C₆ éventuellement substitué par R₄, alkyle en C₁-C₆ éventuellement substitué par R₄, cycloalkyle en C₃-C₆ éventuellement substitué par R₄, alcényle en C₂-C₆ éventuellement substitué par R₄, alcynyle en C₂-C₆ éventuellement substitué par R₄, aryle éventuellement substitué par R₄, ou un hétérocycle aromatique de 5 ou 6 chaînons contenant 1-4 hétéroatomes choisis parmi N et O, lié par un carbone ou un azote;
R₅ est alkyle en C₁-C₆, alcényle en C₂-C₆, alcynyle en C₂-C₆, cycloalkyle en C₃-C₆, aryle ou hétéroaryle;
chacun des R₆ (qui peuvent être identiques ou différents) est H, alkyle en C₁-C₆, alcényle en C₂-C₆, alcynyle en C₂-C₆, cycloalkyle en C₃-C₆, aryle ou hétéroaryle; et
R₇ est aryle ou hétéroaryle;
où "aryle" est un groupe phényle ou naphtyle éventuellement substitué;
"carbocyclique" est un fragment alicyclique saturé de 5 ou 6 atomes de carbone;
"hétérocyclique" est un fragment hétérocyclique saturé de 5 ou 6 atomes comprenant un ou plusieurs N, O et S; et
"hétéroaromatique" est un groupe aromatique de 5 ou 6 atomes dont au moins l'un est N, O ou S;
à condition que R₂ et R₃ ne soient pas tous les deux un hydrogène;
ou un de ses sels pharmaceutiquement acceptables.

2. Composé selon la revendication 1, dans lequel R₄ est présent et est CN, OR₇, SO₂N(R₆)₂, COR₆, CO₂R₆, CON(R₆)₂, NR₁COR₅, NR₁SO₂R₅, NR₁CON(R₆)₂, O-alkyle en C₁-C₆ substitué par R₄, alkyle en C₁-C₆ substitué par R₄, cycloalkyle en C₃-C₆ éventuellement substitué par R₄, alcényle en C₂-C₆ éventuellement substitué par R₄, alcynyle en C₂-C₆ éventuellement substitué par R₄, aryle éventuellement substitué par R₄, ou un hétérocycle aromatique de 5 ou 6 chaînons contenant 1-4 hétéroatomes choisis parmi N et O, lié par un carbone ou un azote.

3. Composé selon la revendication 2, dans lequel R₄ est CN, CON(R₆)₂, cycloalkyle ou aryle éventuellement substitué, ou un hétérocycle aromatique de 5 ou 6 chaînons.

4. Composé selon l'une quelconque des revendications 1 à 3, dans lequel R₂ est halogène, CN, CF₃, alkyle en C₁-C₆ ou OR₁.

5. Composé selon l'une quelconque des revendications 1 à 3, dans lequel R₂ et R₃ forment un cycle.

6. Composé selon la revendication 4, dans lequel R₂ ou R₃ est OR₁.

7. Composé selon la revendication 1, choisi parmi
la 9-méthoxy-5-méthyl-1-(3-méthoxy)phényl-1,3,4,6-tétrahydro-5H-benz[f]-2,5-oxazocine (24a)
la 8-méthoxy-5-méthyl-1-(3-méthoxy)phényl-1,3,4,6-tétrahydro-5H-benz[f]-2,5-oxazocine (24b)
la 9-méthoxy-5-méthyl-1-(4-méthoxy)phényl-1,3,4,6-tétrahydro-5H-benz[f]-2,5-oxazocine (31a) et
la 8-méthoxy-5-méthyl-1-(4-méthoxy)phényl-1,3,4,6-tétrahydro-5H-benz[f]-2,5-oxazocine (31 b).

8. Composé selon la revendication 1, choisi parmi
la 9-cyano-5-méthyl-1-(3-méthoxy)phényl-1,3,4,6-tétrahydro-5H-benz[f]-2,5-oxazocine (22a) et
la 8-cyano-5-méthyl-1-(3-méthoxy)phényl-1,3,4,6-tétrahydro-5H-benz[f]-2,5-oxazocine (22b).

9. Composé selon la revendication 1, choisi parmi
la 9-cyclopropyl-5-méthyl-1-(3-méthoxy)phényl-1,3,4,6-tétrahydro-5H-benz[f]-2,5-oxazocine (29a)
la 8-cyclopropyl-5-méthyl-1-(3-méthoxy)phényl-1,3,4,6-tétrahydro-5H-benz[f]-2,5-oxazocine (29b)
la 9-cyclopropyl-5-méthyl-1-(4-méthoxy)phényl-1,3,4,6-tétrahydro-5H-benz[f]-2,5-oxazocine (30a)
la 8-cyclopropyl-5-méthyl-1-(4-méthoxy)phényl-1,3,4,6-tétrahydro-5H-benz[f]-2,5-oxazocine (30b)
la 9-cyano-5-méthyl-1-(4-méthoxy)phényl-1,3,4,6-tétrahydro-5H-benz[f]-2,5-oxazocine (32a)
la 9-cyano-5-méthyl-1-phényl-1,3,4,6-tétrahydro-5H-benz[f]-2,5-oxazocine (6a)
la 8-cyano-5-méthyl-1-phényl-1,3,4,6-tétrahydro-5H-benz[f]-2,5-oxazocine (6b)
le 5-méthyl-1-phényl-1,3,4,6-tétrahydro-5H-benz[f]-2,5-oxazocine-9-carboxamide (7a)
le 5-méthyl-1-phényl-1,3,4,6-tétrahydro-5H-benz[f]-2,5-oxazocine-8-carboxamide (7b)
la N-(1,1,1-triméthylméthoxycarbonyl)-5-méthyl-1-phényl-1,3,4,6-tétrahydro-5H-benz[f]-2,5-oxazocine-9-méthylamine (8a)
la N-(1,1,1-triméthylméthoxycarbonyl)-5-méthyl-1-phényl-1,3,4,6-tétrahydro-5H-benz[f]-2,5-oxazocine-8-méthylamine (8b)
la 5-méthyl-1,9-diphényl-1,3,4,6-tétrahydro-5H-benz[f]-2,5-oxazocine (9a)
la 5-méthyl-1,8-diphényl-1,3,4,6-tétrahydro-5H-benz[f]-2,5-oxazocine (9b)
la 9-(3,5-diméthylisoxazol-4-yl)-5-méthyl-1-phényl-1,3,4,6-tétrahydro-5H-benz[f]-2,5-oxazocine (10a)
la 8-(3,5-diméthylisoxazol-4-yl)-5-méthyl-1-phényl-1,3,4,6-tétrahydro-5H-benz[f]-2,5-oxazocine (10b)
le 2-(5-méthyl-1-phényl-1,3,4,6-tétrahydro-5H-benz[f]-2,5-oxazocine-9-éthényl)carboxamide (11a)
le 2-(5-méthyl-1-phényl-1,3,4,6-tétrahydro-5H-benz[f]-2,5-oxazocine-8-éthényl)carboxamide (11b)
le 2-(5-méthyl-1-phényl-1,3,4,6-tétrahydro-5H-benz[f]-2,5-oxazocine-9-éthyl)carboxamide (12a)
le 2-(5-méthyl-1-phényl-1,3,4,6-tétrahydro-5H-benz[f]-2,5-oxazocine-8-éthyl)carboxamide (12b)
la 5-méthyl-1-phényl-1,3,4,6-tétrahydro-5H-benz[f]-2,5-oxazocine-9-méthylamine (13a)
la 5-méthyl-1-phényl-1,3,4,6-tétrahydro-5H-benz[f]-2,5-oxazocine-8-méthylamine (13b) et
la N-(acétyl)-5-méthyl-1-phényl-1,3,4,6-tétrahydro-5H-benz[f]-2,5-oxazocine-9-méthylamine (27a).

10. Composé selon la revendication 1, choisi parmi
la 9-bromo-5-méthyl-1-(4-méthoxyphényl)-1,3,4,6-tétrahydro-5H-benz[f]-2,5-oxazocine (17a)
la 8-bromo-5-méthyl-1-(4-méthoxyphényl)-1,3,4,6-tétrahydro-5H-benz[f]-2,5-oxazocine (1 7b)
la 9-bromo-5-méthyl-1-(3-méthoxyphényl)-1,3,4,6-tétrahydro-5H-benz[f]-2,5-oxazocine (21a) et
la 8-bromo-5-méthyl-1-(3-méthoxyphényl)-1,3,4,6-tétrahydro-5H-benz[f]-2,5-oxazocine (21 b).

11. Composition pharmaceutique à utiliser en thérapie, comprenant un composé selon l'une quelconque des revendications 1 à 10, et un diluant ou support pharmaceutiquement acceptable.

12. Utilisation d'un composé selon l'une quelconque des revendications 1 à 10 pour la fabrication d'un médicament humain ou vétérinaire destiné au traitement ou à la prévention d'une maladie choisie parmi la dépression, les troubles de stress post-traumatique, les troubles de déficit de l'attention, les troubles obsessionnels compulsifs, le syndrome prémenstruel, la toxicomanie, les troubles de la miction et le dysfonctionnement sexuel.

13. Utilisation d'un composé selon l'une quelconque des revendications 1 à 10 pour la fabrication d'un médicament humain ou vétérinaire destiné au traitement d'une douleur aiguë, chronique ou névropathique.

14. Utilisation selon la revendication 13, dans laquelle la douleur est associée à un cancer, une opération chirurgicale, une arthrite, une chirurgie dentaire, des névropathies douloureuses, un traumatisme, une lésion ou une maladie musculo-squelettique ou une maladie viscérale, ou à un mal de tête migraineux.

15. Utilisation selon la revendication 13 ou la revendication 14, dans laquelle le sujet est aussi traité avec un opiacé.

16. Utilisation selon la revendication 13 ou la revendication 14, dans laquelle le sujet est aussi traité avec un inducteur d'analgésie choisi parmi l'acétaminophène, un médicament anti-inflammatoire non stéroïdien, un analgésique narcotique, un anesthésique local, un antagoniste de NMDA, un agent neuroleptique, un anticonvulsivant, un antispasmodique, un antidépresseur ou un myorelaxant.

17. Utilisation d'un composés selon l'une quelconque des revendications 1 à 10 pour la fabrication d'un médicament humain ou vétérinaire destiné au traitement ou à la prévention des vomissements.

18. Utilisation selon la revendication 17, dans laquelle les vomissements sont des vomissements aigus, retardés, post-opératoires, de phase terminale ou anticipatoires.

19. Utilisation selon la revendication 17, dans laquelle les vomissements sont induits par une chimiothérapie, des rayonnements, des toxines, une grossesse, des troubles vestibulaires, les transports, des nausées post-opératoires, une opération chirurgicale, une obstruction gastro-intestinale, une motricité gastro-intestinale réduite, une douleur viscérale, une migraine ou des analgésiques opioïdes.
